# EUROPEAN PATENT SPECIFICATION

(11) **EP 2 027 108 B1**
(45) Date of publication and mention of the grant of the patent: **13.01.2010**
(21) Application number: 07729255.5
(22) Date of filing: 17.05.2007
(51) Int. Cl.: C07D 401/12, A61K 31/4545, A61P 11/00, A61P 37/08

(54) **HISTAMINE RECEPTOR ANTAGONISTS COMPRISING AN AZEPIN CORE**
EINEN AZEPIN-KERN ENTHALTENDE ANTAGONISTEN DES HISTAMINREZEPTORS
ANTAGONISTES DE RÉCEPTEUR D'HISTAMINE COMPRENANT UN NOYAU D'AZEPINE

(30) Priority: 18.05.2006 GB 0609897
(43) Date of publication of application: 25.02.2009
(73) Proprietor: Glaxo Group Limited, Greenford Middlesex UB6 0NN (GB)
(72) Inventor: ANCLIFF, Rachael Ann, Stevenage Hertfordshire SG1 2NY (GB); HANCOCK, Ashley Paul, Stevenage Hertfordshire SG1 2NY (GB); KRANZ, Michael Joachim, Stevenage Hertfordshire SG1 2NY (GB); PARR, Nigel, James, Stevenage Hertfordshire SG1 2NY (GB)
(74) Representative: Priestley, Ruth Elizabeth
(86) International application number: PCT/EP2007/054807
(87) International publication number: WO 2007/135081

(56) References cited:
- WO-A-2004/035556

## Description

### COMPOUNDS

The present invention relates to compounds, pharmaceutical compositions containing them and to their use in the treatment of various diseases, in particular inflammatory and/or allergic diseases of the respiratory tract.

Allergic rhinitis, pulmonary inflammation and congestion are medical conditions that are often associated with other conditions such as asthma, chronic obstructive pulmonary disease (COPD), seasonal allergic rhinitis and perennial allergic rhinitis, In general these conditions are mediated, at least in part, by inflammation associated with the release of histamine from various cells, in particular mast cells.

Allergic rhinitis, also known as 'hay fever', affects a large proportion of the population worldwide.
There are two types of allergic rhinitis; seasonal and perennial. The clinical symptoms of seasonal allergic rhinitis typically include nasal itching and irritation, sneezing and watery rhinorrhea which is often accompanied by nasal congestion. The clinical symptoms of perennial allergic rhinitis are similar except that nasal blockage may be more pronounced. Either type of allergic rhinitis may also cause other symptoms such as itching of the throat and/or eyes, epiphora and oedema around the eyes. The symptoms of allergic rhinitis may vary in intensity from the nuisance level to debilitating.

Allergic rhinitis and other allergic conditions are associated with the release of histamine from various cell types, but particularly mast cells. The physiological effects of histamine are classically mediated by three receptor subtypes, termed H1, H2 and H3. H1 receptors are widely distributed throughout the CNS and periphery, and are involved in wakefulness and acute inflammation. H2 receptors mediate gastric acid secretion in response to histamine. H3 receptors are present on the nerve endings in both the CNS and periphery and mediate inhibition of neurotransmitter release [Hill et al., Pharmacol. Rev., 49:253-278, (1997)]. Recently, a fourth member of the histamine receptor family has been identified, termed the H4 receptor [Hough, Mol. Pharmacol., 59:415-419, (2001)].
Whilst the distribution of the H4 receptor appears to be restricted to cells of the immune and inflammatory systems, a physiological role for this receptor remains to be clarified.

The activation of H1 receptors in blood vessels and nerve endings is responsible for many of the symptoms of allergic rhinitis, which include itching, sneezing, and the production of watery rhinorrhea. Oral antihistamine compounds (such as chlorphenyramine, cetirizine, desloratidine and fexofenadine) and intranasal antihistamines (such as azelastine and levocabastine) which are selective H1 receptor antagonists are effective in treating the itching, sneezing and rhinorrhea associated with allergic rhinitis, but are not effective against the nasal congestion symptoms [Aaronson, Ann. Allergy, 67;541-547, (1991)]. Thus, H1 receptor antagonists have been administered in combination with sympathomimetic agents such as pseudoephedrine or oxymetazoline to treat the nasal congestion symptoms of allergic rhinitis. These drugs are thought to produce a decongestant action by activating α-adrenergic receptors and increasing the vascular tone of blood vessels in the nasal mucosa. The use of sympathomimetic drugs for the treatment of nasal congestion is frequently limited by the CNS stimulant properties and their effects on blood pressure and heart rate. A treatment which decreases nasal congestion without having effects on the CNS and cardiovascular system may therefore offer advantages over existing therapies.

Histamine H3 receptors are expressed widely on both CNS and peripheral nerve endings and mediate the inhibition of neurotransmitter release. *In vitro* electrical stimulation of peripheral sympathetic nerves in isolated human saphenous vein results in an increase in noradrenaline release and smooth muscle contraction, which can be inhibited by histamine H3 receptor agonists [Molderings et a/.. Naunyn-Schmiedeberg's Arch. Pharmacol., 346:46-50, (1992); Valentine et al., Eur. J. Pharmacol., 366:73-78, (1999)]. H3 receptor agonists also inhibit the effect of sympathetic nerve activation on vascular tone in porcine nasal mucosa [Varty & Hey., Eur. J. Pharmacol., 452:339-345, (2002)]. *In vivo,* H3 receptor agonists inhibit the decrease in nasal airway resistance produced by sympathetic nerve activation [Hey et al., Arzneim-Forsch Drug Res., 48:881-888, (1998)]. Activation of histamine H3 receptors in human nasal mucosa inhibits sympathetic vasoconstriction [Varty et al., Eur. J. Pharmacol., 484:83-89, (2004)]. Furthermore, H3 receptor antagonists, in combination with histamine H1 receptor antagonists, have been shown to reverse the effects of mast cell activation on nasal airway resistance and nasal cavity volume, an index of nasal congestion [Mcleod at al., Am. J. Rhinol., 13:391-399, (1999)], and further evidence for the contribution of H3 receptors to histamine-induced nasal blockage is provided by histamine nasal challenge studies performed on normal human subjects [Taylor-Clark et al., Br. J. Pharmacol., 144, 867-874, (2005)], although the H3 mechanism in this regard would appear to be novel and unprecedented and may ultimately prove to be clinically silent.

WO2004/035556 discloses substituted piperazines, (1,4) diazepines and 2,5-diazabicyclo [2.2.1] heptanes as histamine H3 or histamine H1/H3 dual antagonists or reverse agonists.

A novel class of compounds has been found that are dual histamine H1 and H3 receptor antagonists. By 'dual' histamine H1 and H3 receptor antagonists it is meant that compounds have activity at both receptor subtypes. For example, the activity at the H1 receptor may be within approximately 100 fold of the activity at the H3 receptor, such as within approximately 10 fold or less.

There is thus provided, in a first aspect of the present invention a compound of formula (I) 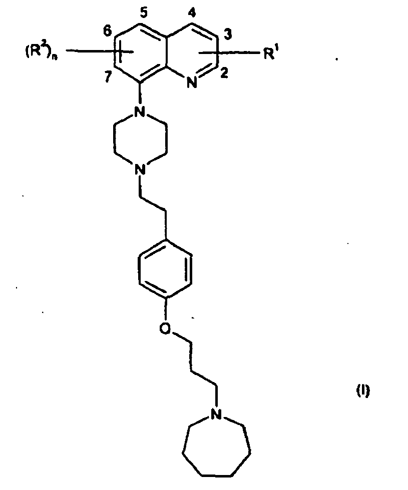 wherein
the quinolinyl ring can be substituted in the 2 or 3 position by R¹ and in the 5, 6 or 7 position by R²;
R¹ represents -CH₂CH₂COOH, -CH=C(CH₃)COOH, or -CH=CHCOOH;
R² represents C₁₋₆ alkyl, aryl or -C₁₋₆alkylaryl;
n represents 0 or 1;
   or a salt thereof.

The compounds of formula (I) may be expected to be useful in the treatment of various diseases in particular inflammatory and/or allergic diseases, such as inflammatory and/or allergic diseases of the respiratory tract, for example allergic rhinitis, that are associated with the release of histamine from cells such as mast cells. Further, the compounds of formula (I) may show an improved profile in that they may possess one or more of the following properties:
(i) H3 antagonist and/or inverse agonist activity with a pKi (pKb) of greater than about 7, for example greater than about 8;
(ii) H1 receptor antagonist and/or inverse agonist activity with a pKi (pKb) of greater than 7, for example greater than about 8;
(iii) lower CNS penetration.

Compounds having such a profile may be suitable for intranasal delivery, and/or capable of once daily administration and/or further may have an improved side effect profile compared with other existing therapies.

In another aspect, there is provided a compound of formula (I) wherein the quinolinyl ring is substituted in the 2 position by R¹.

In another aspect, there is provided a compound of formula (I) wherein R¹ represents - CH₂CH₂COOH.

In another aspect, there is provided a compound of formula (I) wherein the quinolinyl ring is substituted in the 6 position by R²

In another aspect, there is provided a compound of formula (I) wherein R² represents C₁₋₄alkyl (e.g. methyl or ethyl), phenyl, or -C₁₋₄alkylphenyl (e.g. -methylphenyl).

In another aspect, n is 1.

In another aspect, there is provided a compound which is 3-[8-{4-[2-(4-{[3-(hexahydro-1*H*-azepin-1-yl}propyl]oxy}phenyl)ethyl]-1-piperazinyl}-6-(phenylmethyl)-2-quinolinyl]propanoic acid or a salt thereof, particularly a pharmaceutically acceptable salt thereof.

Representative compounds include but are not limited to Examples 1 to 6 described herein, and salts thereof.

It is to be understood that the invention includes all possible combinations of groups, aspects and substituents described herein.

C₁₋₆alkyl, whether alone or as part of another group, may be straight chain or branched. Representative examples include methyl, ethyl, n-propyl, iso-propyl, n-butyl, sec-butyl, iso-butyl, t-butyl, n-pentyl, neo-pentyl and n-hexyl. Particular alkyl groups are C₁₋₄ alkyl e.g. methyl, ethyl, propyl and butyl, such as methyl and ethyl.

It is to be understood that the present invention covers compounds of formula (I) as the free base and as salts thereof, for example as a pharmaceutically acceptable salt.

It is to be further understood that references hereinafter to compounds of the invention or to compounds of formula (I) mean a compound of formula (I) as the free base, or as a salt, or as a solvate.

Because of their potential use in medicine, salts of the compounds of formula (I) are desirably pharmaceutically acceptable. Suitable pharmaceutically acceptable salts can include acid or base addition salts. As used herein, the term 'pharmaceutically acceptable salt' means any pharmaceutically acceptable salt or solvate of a compound of the invention, which upon administration to the recipient is capable of providing (directly or indirectly). For a review on suitable salts see Berge et al., J. Pharm. Sci., 66:1-19, (1977). Typically, a pharmaceutically acceptable salt may be readily prepared by using a desired acid or base as appropriate. The resultant salt may precipitate from solution and be collected by filtration or may be recovered by evaporation of the solvent

A pharmaceutically acceptable base addition salt can be formed by reaction of a compound of formula (I) with a suitable inorganic or organic base, (e.g. triethylamine, ethanolamine, triethanolamine, choline, arginine, lysine or histidine), optionally in a suitable solvent, to give the base addition salt which is usually isolated, for example, by crystallisation and filtration.

Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

A pharmaceutically acceptable acid addition salt can be formed by reaction of a compound of formula (I) with a suitable inorganic or organic acid (such as hydrobromic, hydrochloric, sulphuric, nitric, phosphoric, succinc, maleic, formic, acetic, propionic, fumaric, citric, tartaric, lactic, benzoic, salicylic, glutamaic, aspartic, p-toluenesulfonic, benzenesulfonic, methanesulfonic, ethanesulfonic, naphthalenesulfonic such as 2-naphthalenesulfonic, or hexanoic acid), optionally in a suitable solvent such as an organic solvent, to give the salt which is usually isolated for example by crystallisation and filtration. A pharmaceutically acceptable acid addition salt of a compound of formula (I) can comprise or be for example a hydrobromide, hydrochloride, sulfate, nitrate, phosphate, succinate, maleate, formate, acetate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2-naphthalenesulfonate) or hexanoate salt.

In another aspect there is provided a compound of formula (I) or a formate salt thereof.

Other non-pharmaceutically acceptable salts, e.g. oxalates or trifluoroacetates, may be used, for example in the isolation of the compounds of formula (I), and are included within the scope of this invention.

The invention includes within its scope all possible stoichiometric and non-stoichiometric forms of the salts of the compounds of formula (I).

It will be appreciated that many organic compounds can form complexes with solvents in which they are reacted or from which they are precipitated or crystallized. These complexes are known as "solvates". For example, a complex with water is known as a "hydrate". Solvents with high boiling points and/or capable of forming hydrogen bonds such as water, xylene, N-methyl pyrrolidinone, methanol and ethanol may be used to form solvates. Methods for identification of solvates include, but are not limited to, NMR and microanalysis. Solvates of the compounds of formula (I) are within the scope of the invention.

The compounds of formula (I) may be in crystalline or amorphous form. Furthermore, some of the crystalline forms of the compounds of formula (I) may exist as polymorphs, which are included within the scope of the present invention. The most thermodynamically stable polymorphic form of compounds of formula (I) are of particular interest

Polymorphic forms of compounds of formula (I) may be characterized and differentiated using a number of conventional analytical techniques, including, but not limited to, X-ray powder diffraction (XRPD) patterns, infrared (IR) spectra, Raman spectra, differential scanning calorimetry (DSC), thermogravimetric analysis (TGA) and solid state nuclear magnetic resonance (NMR).

Further it will be understood that the present invention encompasses geometric isomers of the compounds of formula (I) including cis and trans configurations, and regioisomers including exo and endo double bonds (e.g. -CH=C(CH₃)CO₂H and -CH-C(=CH₂)CO₂H), whether as individual isomers isolated such as to be substantially free of the other isomers (i.e. pure) or as mixtures thereof. Thus, for example the present invention encompases an individual isomer isolated such, as to be substantially free of the other isomer (i.e.pure) such that less than 10%, for example less than 1% or less than 0.1 % of the other isomer is present Separation of geometric isomers may be achieved by conventional techniques, e.g. by fractional crystallisation, chromatography or HPLC.

Certain compounds of formula (I) may exist in one of several tautomeric forms. It will be understood that the present invention encompasses all tautomers of the compounds of formula (I) whether as individual tautomers or as mixtures thereof.

It will be appreciated from the foregoing that included within the scope of the invention are solvates, hydrates, complexes, isomers and polymorphic forms of the compounds of formula (I) and salts thereof.

There is disclosed herein processes for the preparation of compounds of formula (I) or salts thereof.

According to a first process A, a compound of formula (I) may be prepared by deprotection of a compound of formula (Ia): 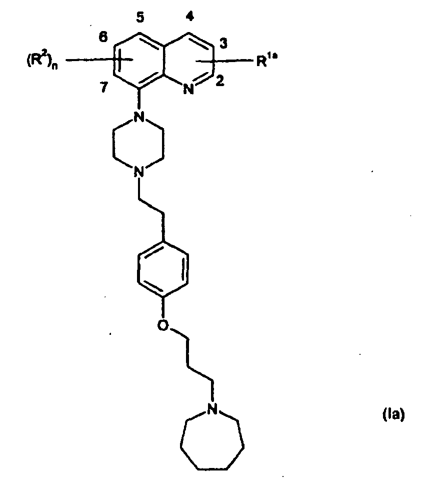 wherein
the quinolinyl ring is substituted in the 2 or 3 position by R^{1a} and in the 5, 6, or 7 position by R²; R^{1a} represents a protected derivative of R¹, such as an ester of R¹, for example, -CH₂CH₂COOR^{x}, - CH=C(CH₃)COOR^{x} or -CH=CHCOOR^{x} in which R^{x} Independently represents a protecting group such as C₁-C₆ alkyl eg. methyl, ethyl or t-butyl, particularly methyl, and isomers thereof; and R² and n are as defined for formula (I) above.

Deprotection may be performed under standard conditions. Thus, hydrolysis of a carboxylic acid ester may be performed in the presence of a suitable base, for example, sodium hydroxide or potassium hydroxide, in a suitable aqueous solvent system such as methanol/water or tetrahydrofuran/water, optionally at an elevated temperature such as reflux. Alternatively, hydrolysis of a carboxylic acid ester, for example, the t-butyl ester may be performed in the presence of a suitable acid such as hydrogen chloride in dioxane under standard conditions for acid hydrolysis.

Compounds of formula (Ia) may be prepared by reacting a compound for formula (II) 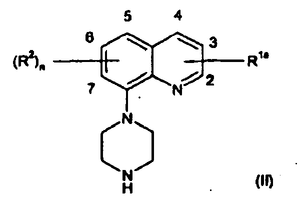 wherein the quinolinyl ring is substituted as defined hereinabove and R^{1a}, R² and n are as defined hereinabove,
with a compound of formula (III) 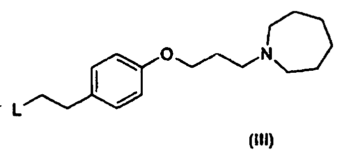 wherein L represents a leaving group such as an activated hydroxyl e.g. mesylate or tosylate. The tertiary amine may be prepared under standard conditions for N-alkylation of a secondary amine such as those described herein, typically in the presence of a base e.g. sodium hydrogen carbonate and a suitable solvent such as acetonitrile at an elevated temperature such as about 80°C.

A compound of formula (III) may be prepared in accordance with the following general reaction scheme:

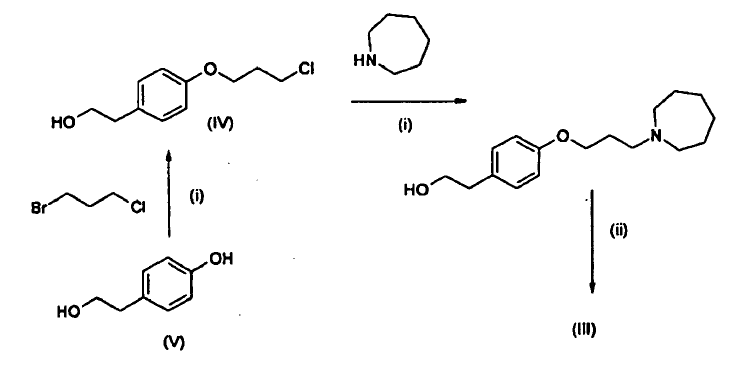

Reagents and reaction conditions: (i) suitable base e.g. potassium carbonate (K₂CO₃), solvent e.g. 2-butanone, usually at an elevated temperature, optionally using microwave irradiation, and optionally with a catalytic amount of activating agent eg potassium iodide; (ii) suitable activating agent eg. tosyl chloride (TsCl) or mesyl chloride (MsCl), an appropriate base eg. diisopropylethylamine (DIPEA), in a suitable solvent, such as dichloromethane (DCM).

1-bromo-3-chloropropane, hexahydro-1*H*-azepine and the compound of formula V (2-(4-hydroxyphenyl)ethanol) are commercially available, for example, from Aldrich.

A compound of formula (II) may be prepared according to the following reaction schemes (Schemes 1 to 3): 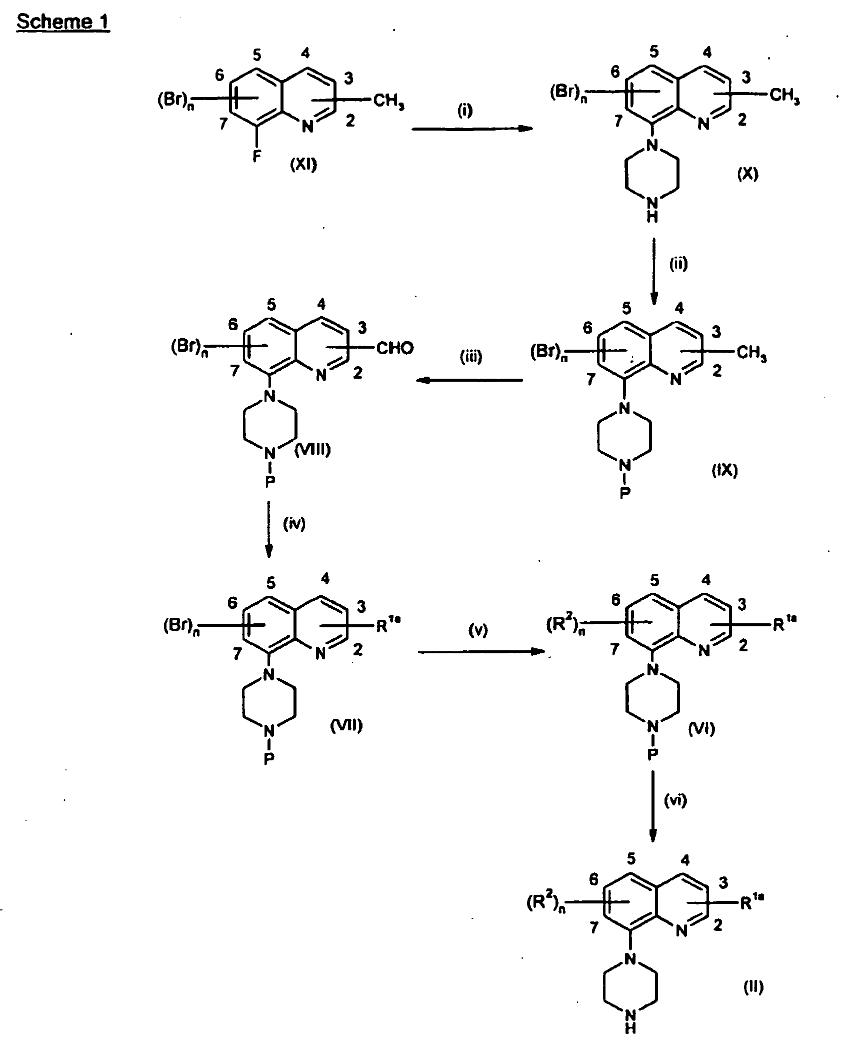 wherein the quinolinyl ring is substituted in the 3 position by CH₃, and when n is 1, in the 5, 6, or 7 position by Br, R^{1a} is as defined hereinabove and P represents a protecting group such as benzylcarbamate (cbz) or di-*tert*-butyl dicarbonate (BOC).

**Reagents and reaction conditions:** (i) reaction with piperazine in a suitable solvent e.g. dimethylsuphoxide in the presence of a suitable base such as potassium carbonate, at an elevated temperature e.g. about 100°C to about 150°C; (ii) protection with a suitable protecting group such as cbz or BOC using benzylchloro formate or di-*tert*-butyl dicarbonate, typically in the presence of a suitable base such as triethylamine and/or dimethylaminopyridine, in an appropriate solvent such as dichloromethane (DCM); (iii) oxidation with a suitable oxidant such as selenium dioxide, in an appropriate solvent such as dioxane, at an elevated temperature such as about 55 °C to about 80 °C, under nitrogen; (iv) Wittig reaction with a suitable phosphorus ylid such as methyl(biphenylphosphoranylidene) acetate or carboethoxymethylenetriphenylphosphorane in a suitable solvent such as tetrahydrofuran (THF), at an elevated temperature such as about 65 °C; (v) alkylation with an appropriate alkylboron such as triethylborane or *B*-Benzyl-9-borabicyclononane, typically under palladium catalysis, in the presence of a suitable base e.g. potassium carbonate, in a suitable solvent such as DMF, optionally at an elevated temperature, or in alternative method from the appropriate Grignard reagent in the presence of an iron catalyst such as iron (III) acetoacetate (Fe(acac)₃) in a solvent such as THF:*N*-methyl pyrrolidinone (9:1), typically between 5 °C and 20 °C: (vi) deprotection under standard conditions such as in the presence of a suitable acid such as trifluoroacetic acid or in the case of BOC deprotection, hydrogen chloride in dioxane.

Compounds of formula (XI) may be made from known or commercially available materials. Compounds of formula (XI) in which the methyl group is in the 2 position may be prepared by Skraup quinoline synthesis typically involving reaction with crotonaldehyde and the appropriate aniline, in the presence of a strong acid such as 5M hydrogen chloride, in a suitable solvent e.g. toluene at an appropriate elevated temperature such as at about 100 °C. Available anilines include 4-bromo-2-fluoroaniline and 5-bromo-2-fluoroaniline which are commercially available, for example from Fluorochem. 3-Bromo-2-fluoronitrobenzene is disclosed in Acta Chem. Scand. Series B, 1975, B29, 981-2, which may be converted to 3-bromo-2-fluoraniline by reduction of the nitro group using methods well known to those skilled in the art, for example, using an appropriate reducing agent, such as tin chloride or iron, with a suitable acid, for example hydrochloric acid.

Compounds of formula (VII) in scheme 1, in which n is 0 may also be prepared according to the following reaction scheme (Scheme 2): 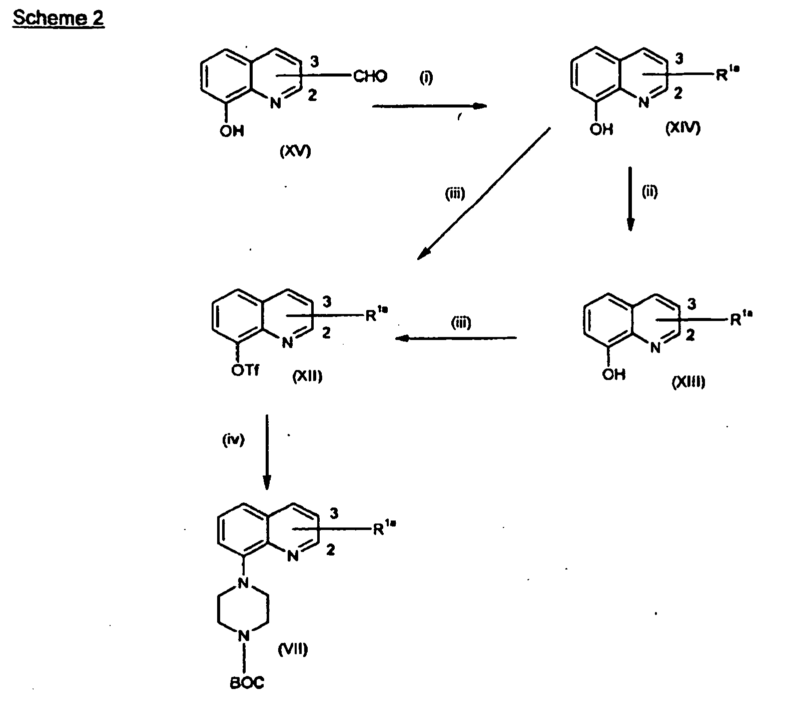 wherein the quinolinyl ring is substituted on the 2 or 3 position by CHO or R^{1a} as indicated and R^{1a} is as defined hereinabove.

**Reagents and reaction conditions:** (i) Wittig reaction using a suitable ylid such as those described above, in a suitable solvent such as toluene, optionally at an elevated temperature e.g. about 50°C; (ii) optional hydrogenation in the presence of 10% w/w palladium on carbon in a suitable solvent such as ethylacetate to give compounds in which R^{1a} represents -CH₂-CH₂COOR^{x} in which R^{x} is as defined hereinabove; (iii) activation with a suitable activating agent e.g. *N*-phenyl bis(trifluoromethanesulfonamide, in the presence of suitable base e.g. triethylamine, in a suitable solvent such as DMF, or with triftic anhydride in the presence of pyridine at a reduced temperature e.g. about 0°C; (iv) alkylation with 1,1-dimethylethyl 1-piperazinecarboxylate, caesium carbonate, tris(dibenzylideneacetone) dipalladium (0), 2-(dicyclohexylphosphino)-2'-(*N,N-*dimethylamino)biphenyl in the presence of a suitable solvent, e.g. THF, optionally at an elevated temperature.

Compounds of formula (XV), such as 8-hydroxyquinoline-2-carboxaldehyde is commercially available from Acros or may be prepared from known materials. Thus, for example, the compound of formula (XV) in which the aldehyde group is in the 3 position may be prepared from the corresponding methylquinoline, which is disclosed in Tetrahedron, 1996, 52, 2937-2944 (see scheme 2, compound 2a), by oxidation with an appropriate oxidising agent, such as selenium dioxide.

It will be appreciated that a compound of formula (VII) may also be prepared by interconversion from other compounds of formula (VII) using conventional interconvension procedure such as hydrogenation under standard conditions as described herein, and by isomerisation of geometric isomers also as described herein.

Compounds of formula (VII) in scheme 1, in which n is 0 may also be prepared according to the following reaction scheme (Scheme 3): 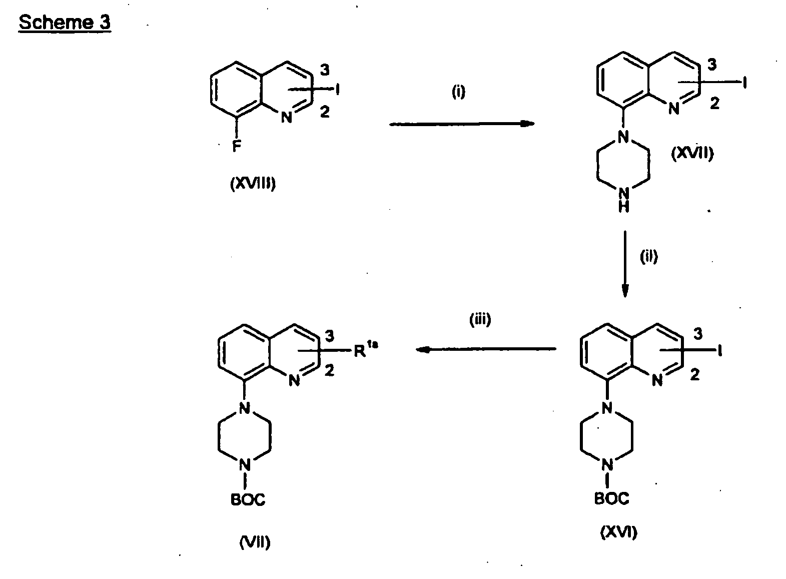 wherein the quinolinyl ring is substituted in the 3 position by I or R^{1a} as indicated and R^{1a} is as described hereinabove.

**Reagents and reaction conditions:** (i) reaction with piperazine, in a suitable solvent e.g. dimethylsuphoxide, in the presence of a suitable base such as potassium carbonate, at an elevated temperature such as about 100°C to about 150°C; (ii) protection with a suitable protecting group such as BOC using di-*tert*-butyl dicarbonate, typically in the presence of a suitable base such as triethylamine and/or dimethylaminopyridine, in an appropriate solvent such as DCM; (iii) Heck reaction with an acrylate ester such as methyl methacrylate, ethyl methacrylate or *t*-butyl methacrylate, suitable base such as triethylamine, a phosphine such as triphenylphosphine, a suitable catalyst such as palladium (II) acetate, in a suitable solvent such as DMF, at an elevated temperature, for example about 100°C.

The compound of formula (XVIII) in which iodine is substituted in the 3 position (i.e. 8-fluoro-3-iodoquinoline) is disclosed in International Patent Application WO05/095346 (see Description 1, page 12) and/or is disclosed in International Patent Application WO2007039220A1 (see page 16, Description 1).

Acrylate esters are known and/or commercially available. Thus, for example, methyl acrylate, methyl methacrylate, ethyl methacylate and *t*-butyl methacrylate are available from Aldrich and/or Acros.

In an alternative method, compounds of formula (Ia) wherein n is 0 may be prepared by reacting a compound of formula (XII) or a compound of formula (XIX) 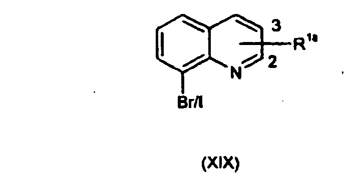 wherein the quinolinyl ring is substituted in the 2 or 3 position by R^{1a} with a compound of formula (XX)

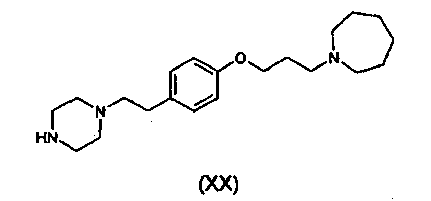

The Büchwald reaction typically takes place in the presence of a suitable base e.g. caesium carbonate, a palladium catalyst such as tris(dibenzylideneacetone) dipalladium (0), a ligand such as 2-(dicydohexylphosphino)-2'-(*N*,*N*-dimethylamino)biphenyl, in a suitable solvent such as THF, at an elevated temperature e.g. reflux.

A compound of formula (XIX) may be prepared by a Wittig reaction in which a compound of formula (XXI): 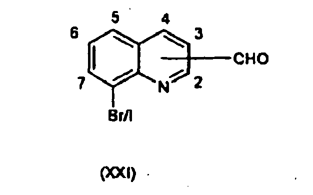 is reacted with an appropriate phosphorus ylid, under similar conditions to the Wittig reaction described above.

Compounds of formula (XXI) may be prepared by oxidation of the appropriate methylquinoline as described herein. Methylquinolines are commercially available and/or known, for example, 8-bromo-2-methylquinoline is available from ACB Blocks or may be prepared according to the methods described by Leir, C. M., J. Org. Chem. 42(5):911-913 (1977) see Table 1. 8-iodo-2-methylquinoline is available from Maybridge and 8-bromo-3-methylquinoline is discloses in International Patent Application W02002010131.

A compound of formula (XX) may be prepared according to the following reaction scheme:

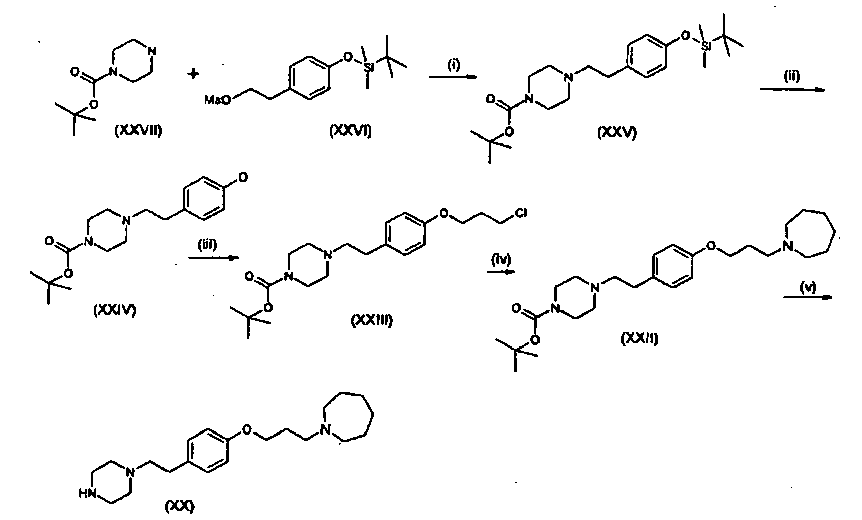

**Reagents and reaction conditions:** (i) base e.g. sodium bicarbonate, solvent e.g. acetonitrile, optionally at an elevated temperature e.g. reflux; (ii) deprotection e.g. in the presence of tetrabutylammonium fluoride in a suitable solvent e.g. tetrahydrofuran; (iii) alkylation with 1-bromo-3-chloropropane in the presence of a suitable base e.g. potassium carbonate, suitable solvent e.g. 2-butanone; (iv) alkylation with homopiperazine in the presence of a suitable base e.g. potassium carbonate, suitable solvent e.g. 2-butanone, optionally in the presence of an activating agent e.g. potassium iodide and optionally at an elevated temperature e.g. reflux; (v) deprotection e.g. in the presence of trifluoracetic acid.

According to a second process (B) compounds of formula (I) may be prepared by
(i) reacting a compound of formula (II) with a compound of formula (III) to form a compound of formula (Ia); and
(ii) deprotecting the compound of formula (Ia) to form a compound of formula (I). In process (B) the intermediate compound of formula (Ia) need not be isolated.

According to a third process, C, a compound of formula (I), may be prepared by interconversion from other compounds of formula (I). Thus, compounds of formula (I) may also be prepared from other compounds of formula (I) using conventional interconversion procedures such as isomerisation of geometric isomers e.g. interconversion between cis and trans isomers and interconversion between an exo and endo double bond, for example, inerconversion between -CH=C(CH₃)COOH and -CH₂-C(=CH₂)COOH. Thus, interconversion from other compounds of formula (I) (process C) forms yet a further aspect of the present invention.

According to a fourth process, D, a salt of a compound of formula (I) may be prepared by exchange of counterions, or precipitation of said salt from the free base.

Examples of protecting groups that may be employed in the synthetic routes described and the means for their removal can be found in T. W. Greene 'Protective Groups in Organic Synthesis' (3rd edition, J. Wiley and Sons, 1999). Suitable amine protecting groups include sulfonyl (e.g. tosyl), acyl (e.g. acetyl, 2',2',2'-trichloroethoxycarbonyl, benzyloxycarbonyl or *t*-butoxycarbonyl) and arylalkyl (e.g. benzyl), which may be removed by hydrolysis (e.g. using an acid such as hydrogen chlorine in dioxane or trifluoroacetic acid in dichloromethane) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃), which may be removed by base catalysed hydrolysis or a solid phase resin bound benzyl group, such as a Merrifield resin bound 2,6-dimethoxybenzyl group (Ellman linker), which may be removed by acid cleavage, for example with trifluoroacetic acid.

Examples of disease states in which a compound of formula (I), or a pharmaceutically acceptable salt thereof may have potentially beneficial anti-inflammatory and/or anti-allergic effects include inflammatory and/or allergic diseases of the respiratory tract, such as allergic rhinitis or other diseases such as bronchitis (including chronic bronchitis), asthma (including allergen-induced asthmatic reactions), chronic obstructive pulmonary disease (COPD), sinusitis and allergic rhinitis (seasonal and perennial).

Furthermore, the compounds of formula (I) may be of use in the treatment of nephritis, skin diseases such as psoriasis, eczema, allergic dermatitis and hypersensitivity reactions. Also, the compounds of formula (I) may be useful in the treatment of insect bites and stings.

The compounds of formula (I) may also be of use in the treatment of nasal polyposis, conjunctivitis or pruritis.

A disease of particular interest is allergic rhinitis.

Other diseases in which histamine may have may have a pathophysiological role include non-allegic rhinitis, and also diseases of the gastrointestinal tract such as intestinal inflammatory diseases including inflammatory bowel disease (e.g.Crohn's disease or ulcerative colitis) and intestinal inflammatory diseases secondary to radiation exposure or allergen exposure.

It will be appreciated by those skilled in the art that references herein to treatment or therapy extend to prophylaxis as well as the treatment of established conditions.

As mentioned above, compounds of formula (I) may be useful as therapeutic agents. There is thus provided a compound of formula (I) or a pharmaceutically acceptable salt thereof for use in therapy.

In another embodiment, there is provided a compound which is 3-[8-{4-[2-(4-{[3-(hexahydro-1*H-*azepin-1-yl)propyl]oxy}phenyl)ethyl]-1-piperazinyl}-6-(phenylmethyl)-2-quinolinyl]propanoic acid or a pharmaceutically acceptable salt thereof for use in therapy.

In one embodiment, there is provided the use of a compound of formula (I) or a pharmaceutically acceptable salt thereof for the manufacture of a medicament for the treatment of any of the above diseases.

In another embodiment, there is provided the use of a compound which is 3-[8-{4-[2-(4-{[3-(hexahydro-1*H*-azepin-1-yl)propyl]oxy}phenyl)ethyl]-1-piperazinyl}-6-(phenylmethyl)-2-quinolinyl]propanoic acid or a pharmaceutically acceptable salt thereof for the manufacture of a medicament for the treatment of any of the above diseases.

The disclosure provides a method for the treatment of any of the above diseases, in a human or animal subject in need thereof, which method comprises administering an effective amount of a compound of formula (I) or a pharmaceutically acceptable salt thereof.

The disclosure provides a method for the treatment of any of the above diseases, in a human or animal subject in need thereof, which method comprises administering an effective amount of a compound which is 3-[8-{4-[2-(4-{[3-(hexahydro-1*H*-azepin-1-yl)propyl]oxy}phenyl)ethyl}-1-piperazinyl}-6-(phenylmethyl)-2-quinolinyl]propanoic acid or a pharmaceutically acceptable salt thereof.

When used in therapy, the compounds of formula (I) or pharmaceutically acceptable salts thereof may typically be formulated in a suitable composition. Such compositions may be prepared using standard procedures.

Thus, there is provided a composition which comprises a compound of formula (I) or a pharmaceutically acceptable salt thereof optionally with one or more pharmaceutically acceptable carriers and/or excipients.

There is further provided a composition which comprises a compound which is 3-[8-{4-[2-(4-{[3-(hexahydro-1*H*-azepin-1-yl)propyl]oxy}phenyl)ethyl]-1-piperazinyl}-6-(phenylmethyl)-2-quinolinyl]propanoic acid or a pharmaceutically acceptable salt thereof optionally with one or more pharmaceutically acceptable carriers and/or excipients.

A composition comprising a compound of formula (I) or a pharmaceutically acceptable salt thereof, which may be prepared by admixture, suitably at ambient temperature and atmospheric pressure, may be suitable for topical administration (which includes epicutaneous, inhaled, intranasal or ocular administration), enteral administration (which includes oral or rectal administration) or parenteral administration (such as by injection or infusion). Of interest are compositions comprising a compound of formula (I) or a pharmaceutically acceptable salt thereof, suitable for topical administration, particularly suitable for intranasal administration.

Generally, compositions may be in the form of solutions or suspensions (aqueous or non-aqueous), tablets, capsules, oral liquid preparations, powders, granules, lozenges, lotions, creams, ointments, gels, foams, reconstitutable powders or suppositories as required by the route of administration.

Generally, the compositions comprising a compound of formula (I) or a pharmaceutically acceptable salt thereof may contain from about 0.1 % to 99% (w/w), such as from about 10 to 60% (w/w) (based on the total weight of the composition), of the compound of formula (I) or the pharmaceutically acceptable salt thereof, depending on the route of administration. The dose of the compound used in the treatment of the aforementioned diseases will vary in the usual way with the seriousness of the diseases, the weight of the sufferer, and other similar factors. However, as a general guide, suitable unit doses may be about 0.05 to 1000 mg, for example about 0.05 to 200 mg, and such unit doses may be administered more than once a day, for example two or three times a day or as desired.

Such therapy may extend for a number of weeks or months.

The proportion of the compound of formula (I) or a pharmaceutically acceptable salt thereof in a topical composition will depend on the precise type of composition to be prepared and the particular route of administration, but will generally be within the range of from about 0.001 to 10% (w/w), based on the total weight of the composition. Generally, however for most types of preparations the proportion used will be within the range of from about 0.005 to 1% (w/w), such as about 0.01 to 1% (w/w), for example about 0.01 to 0.5% (wlw), based on the total weight of the composition. However, In powders for inhalation the proportion used will generally be within the range of from about 0.1 to 5% (w/w), based on the total weight of the composition.

Generally, compositions suitable for intranasal or inhaled administration may conveniently be formulated as aerosols, solutions, suspensions, drops, gels or dry powders, optionally with one or more pharmaceutically acceptable carriers and/or excipients such as aqueous or non-aqueous vehicles, thickening agents, isotonicity adjusting agents, antioxidants and/or preservatives.

For compositions suitable for intranasal or inhaled administration, the compound of formula (I) or a pharmaceutically acceptable salt thereof may typically be in a particle-size-reduced form, which may be prepared by conventional techniques, for example, micronisation and milling. Generally, the size-reduced (e.g. micronised) compound of formula (I) or a pharmaceutically acceptable salt thereof can be defined by a D₅₀ value of about 0.5 to 10 microns, such as of about 2 to 4 microns (for example as measured using laser diffraction).

In one aspect, compositions comprising a compound of formula (I) or a pharmaceutically acceptable salt thereof are suitable for intranasal administration. Intranasal compositions comprising a compound of formula (I) or a pharmaceutically acceptable salt thereof may permit the compound(s) to be delivered to all areas of the nasal cavities (the target tissue) and further, may permit the compound(s) to remain in contact with the target tissue for longer periods of time. A suitable dosing regime for intranasal compositions would be for the patient to inhale slowly through the nose subsequent to the nasal cavity being cleared. During inhalation the composition would be administered to one nostril while the other is manually compressed. This procedure would then be repeated for the other nostril. Typically, one or two sprays per nostril would be administered by the above procedure up to two or three times each day, ideally once daily. Of particular interest are intranasal compositions suitable for once daily administration.

Intranasal compositions may optionally contain one or more suspending agents, one or more preservatives, one or more wetting agents and/or one or more isotonicity adjusting agents as desired. Compositions suitable for intranasal administration may optionally further contain other excipients, such as antioxidants (for example sodium metabisulphite), taste-masking agents (such as menthol) and sweetening agents (for example dextrose, glycerol, saccharin and/or sorbitol).

The suspending agent, if included, will typically be present in the intranasal composition in an amount of between about 0.1 and 5% (w/w), such as between about 1.5% and 2.4% (w/w), based on the total weight of the composition. Examples of suspending agents include Avicel®, carboxymethylcellulose, veegum, tragacanth, bentonite, methylcellulose and polyethylene glycols, e.g. microcrystalline cellulose or carboxy methylcellulose sodium. Suspending agents may also be included in compositions suitable for inhaled, ocular and oral administration as appropriate.

For stability purposes, intranasal compositions comprising a compound of formula (I) or a pharmaceutically acceptable salt thereof may be protected from microbial or fungal contamination and growth by inclusion of a preservative. Examples of pharmaceutically acceptable anti-microbial agents or preservatives may include quaternary ammonium compounds (e.g. benzalkonium chloride, benzethonium chloride, cetrimide and cetylpyridinium chloride), mercurial agents (e.g. phenylmercuric nitrate, phenylmercuric acetate and thimerosal), alcoholic agents (e.g. chlorobutanol, phenylethyl alcohol and benzyl alcohol), antibacterial esters (e.g. esters of para-hydroxybenzoic acid), chelating agents such as disodium ethylenediaminetetraacetate (EDTA) and other anti-microbial agents such as chlorhexidine, chlorocresol, sorbic acid and its salts (such as potassium sorbate) and polymyxin. Examples of pharmaceutically acceptable anti-fungal agents or preservatives may include sodium benzoate. The preservative, if included, may be present in an amount of between about 0.001 and 1% (w/w), such as about 0.015% (w/w), based on the total weight of the composition. Preservatives may be included in compositions suitable for other routes of administration as appropriate.

Compositions which contain a suspended medicament may include a pharmaceutically acceptable wetting agent which functions to wet the particles of medicament to facilitate dispersion thereof in the aqueous phase of the composition. Typically, the amount of wetting agent used will not cause foaming of the dispersion during mixing. Examples of wetting agents include fatty alcohols, esters and ethers, such as polyoxyethylene (20) sorbitan monooleate (Polysorbate 80). The wetting agent may be present in intranasal compositions in an amount of between about 0.001 and 0.05% (w/w), for example about 0.025% (w/w), based on the total weight of the composition. Wetting agents may be included in compositions suitable for other routes of administration, e.g. for inhaled and/or ocular administration, as appropriate.

An isotonicity adjusting agent may be included to achieve isotonicity with body fluids e.g. fluids of the nasal cavity, resulting in reduced levels of irritancy. Examples of isotonicity adjusting agents include sodium chloride, dextrose, xylitol and calcium chloride. An isotonicity adjusting agent may be included in intranasal compositions in an amount of between about 0.1 and 10% (w/w), such as about 5.0% (w/w), based on the total weight of the composition. Isotonicity adjusting agents may also be included in compositions suitable for other routes of administration, for example in compositions suitable for inhaled, ocular, oral liquid and parenteral administration, as appropriate.

Further, the intranasal compositions comprising a compound of formula (I) or a pharmaceutically acceptable salt thereof may be buffered by the addition of suitable buffering agents such as sodium citrate, citric acid, phosphates such as disodium phosphate (for example the dodecahydrate, heptahydrate, dihydrate and anhydrous forms) or sodium phosphate and mixtures thereof. Buffering agents may also be included in compositions suitable for other routes of administration as appropriate.

Compositions for administration topically to the nose or lung for example, for the treatment of rhinitis, include pressurised aerosol compositions and aqueous compositions delivered to the nasal cavities by pressurised pump. Compositions which are non-pressurised and adapted to be administered topically to the nasal cavity are of particular interest. Suitable compositions contain water as the diluent or carrier for this purpose. Aqueous compositions for administration to the lung or nose may be provided with conventional excipients such as buffering agents, tonicity modifying agents and the like. Aqueous compositions may also be administered to the nose by nebulisation.

A fluid dispenser may typically be used to deliver a fluid composition to the nasal cavities. The fluid composition may be aqueous or non-aqueous, but typically aqueous. Such a fluid dispenser may have a dispensing nozzle or dispensing orifice through which a metered dose of the fluid composition is dispensed upon the application of a user-applied force to a pump mechanism of the fluid dispenser. Such fluid dispensers are generally provided with a reservoir of multiple metered doses of the fluid composition, the doses being dispensable upon sequential pump actuations. The dispensing nozzle or orifice may be configured for insertion into the nostrils of the user for spray dispensing of the fluid composition into the nasal cavity. A fluid dispenser of the aforementioned type is described and illustrated in WO05/044354 the entire content of which is hereby incorporated herein by reference. The dispenser has a housing which houses a fluid discharge device having a compression pump mounted on a container for containing a fluid composition. The housing has at least one finger-operable side lever which is movable inwardly with respect to the housing to cam the container upwardly in the housing to cause the pump to compress and pump a metered dose of the composition out of a pump stem through a nasal nozzle of the housing. In one embodiment, the fluid dispenser is of the general type illustrated In Figures 30-40 of WO05/044354.

In one aspect, there is provided an intranasal composition comprising a compound of formula (I) or a pharmaceutically acceptable salt thereof. In another aspect, such an intranasal composition is benzalkonium chloride-free.

Inhaled administration involves topical administration to the lung, such as by aerosol or dry powder composition.

Aerosol compositions suitable for inhaled administration may comprise a solution or fine suspension of the compound in a pharmaceutically acceptable aqueous or non-aqueous solvent. Aerosol compositions suitable for inhalation can be either a suspension or a solution and generally contain a compound of formula (I) or a pharmaceutically acceptable salt thereof and a suitable propellant such as a fluorocarbon or hydrogen-containing chlorofluorocarbon or mixtures thereof, such as hydrofluoroalkanes, e.g. 1,1,1,2-tetrafluoroethane, 1,1,1,2,3,3,3-heptafluoro-n-propane or a mixture thereof. The aerosol composition may optionally contain additional excipients well known in the art such as surfactants or cosolvents. Examples of surfactants include, but are not limited to oleic acid, lecithin, an oligolactic acid or derivative e.g. as described in WO94/21229 and WO98/34596. An example of a cosolvent includes, but is not limited to ethanol. Aerosol compositions may be presented in single or multidose quantities in sterile form in a sealed container, which may take the form of a cartridge or refill for use with an atomising device or inhaler. Alternatively, the sealed container may be a unitary dispensing device such as a single dose nasal inhaler or an aerosol dispenser fitted with a metering valve (metered dose inhaler), which is intended for disposal once the contents of the container have been exhausted.

Dry powder inhalable compositions may take the form of capsules and cartridges of, for example, gelatine, or blisters of, for example, laminated aluminium foil, for use in an inhaler or insufflator. Such compositions may be formulated comprising a powder mix of a compound of formula (I) or a pharmaceutically acceptable salt thereof and a suitable powder base such as lactose or starch.

Optionally, for dry powder inhalable compositions, a composition suitable for inhaled administration may be incorporated into a plurality of sealed dose containers (*e.g*. comprising the dry powder composition) mounted longitudinally in a strip or ribbon inside a suitable inhalation device. The container is rupturable or peel-openable on demand and the dose of *e.g*. the dry powder composition may be administered by inhalation via the device such as the DISKUS^{™} device, marketed by GlaxoSmithKline. The DISKUS^{™} inhalation device is for example described in GB 2242134 A, and in such a device, at least one container for the composition in powder form (the container or containers may, for example, be a plurality of sealed dose containers mounted longitudinally in a strip or ribbon) is defined between two members peelably secured to one another; the device comprises: a means of defining an opening station for the said container or containers; a means for peeling the members apart at the opening station to open the container, and an outlet, communicating with the opened container, through which a user can inhale the composition in powder form from the opened container.

Aerosol compositions are typically arranged so that each metered dose or "puff" of aerosol contains about 20 µg - 2000 µg, particularly about 20 µg - 500 µg of a compound of formula (I) or a pharmaceutically acceptable salt thereof. Administration may be once daily or several times daily, for example 2, 3, 4 or 8 times, giving for example 1, 2 or 3 doses each time. The overall daily dose with an aerosol will be within the range of about 100 µg - 10 mg, such as between about 200 µg - 2000 µg. The overall daily dose and the metered dose delivered by capsules and cartridges in an inhaler or insufflator will generally be double those with aerosol compositions.

In another aspect, there is provided a composition comprising a compound of formula (I) or a pharmaceutically acceptable salt thereof which is suitable for epicutaneous administration. An epicutaneous composition to be applied to the affected area e.g. the skin, by one or more application per day, may be in the form of, for example, an ointment, a cream, an emulsion, a lotion, a foam, a spray, an aqueous gel, or a microemulsion. Such compositions may optionally contain one or more solubilising agents, skin-penetration-enhancing agents, surfactants, fragrances, preservatives or emulsifying agents.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, wootfat, beeswax, carboxypolymethylene and cellulose derivatives, and/or glyceryl monostearate and/or non-ionic emulsifying agents. Lotions may be formulated with an aqueous or oily base and will in general also contain one or more emulsifying agents, stabilising agents, dispersing agents, suspending agents or thickening agents.

In another aspect, there is provided a composition comprising a compound of formula (I) or a pharmaceutically acceptable salt thereof which is suitable for ocular administration. Such compositions may optionally contain one or more suspending agents, one or more preservatives, one or more wetting/lubricating agents and/or one or more isotonicity adjusting agents. Examples of ophthalmic wetting/lubricating agents may include cellulose derivatives, dextran 70, gelatin, liquid polyols, polyvinyl alcohol and povidone such as cellulose derivatives and polyols.

In another aspect, there is provided a composition comprising a compound of formula (I) or a pharmaceutically acceptable salt thereof which is suitable for oral administration. Tablets and capsules for oral administration may be in unit dose form, and may contain conventional excipients,
such as binding agents, fillers, tabletting lubricants, disintegrants and acceptable wetting agents. The tablets may be coated according to methods well known In normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

In another aspect, there is provided a composition comprising a compound of formula (I) or a pharmaceutically acceptable salt thereof which is suitable for parenteral administration. Fluid unit dosage forms suitable for parenteral administration may be prepared utilising a compound of formula (I) or pharmaceutically acceptable salt thereof and a sterile vehicle which may be aqueous or oil based. The compound, depending on the vehicle and concentration used, may be either suspended or dissolved in the vehicle. In preparing solutions, the compound may be dissolved for injection and filter sterilised before filling into a suitable vial or ampoule and sealing. Optionally, adjuvants such as a local anaesthetic, preservatives and buffering agents may be dissolved in the vehicle. To enhance the stability, the composition may be frozen after filling into the vial and the water removed under vacuum. The lyophilised parenteral composition may be reconstituted with a suitable solvent just prior to administration. Parenteral suspensions may be prepared in substantially the same manner, except that the compound is suspended in the vehicle instead of being dissolved, and sterilisation cannot be accomplished by filtration. The compound may be sterilised by exposure to ethylene oxide before suspension in a sterile vehicle. A surfactant or wetting agent may be included in the composition to facilitate uniform distribution of the compound.

Pharmaceutical compositions containing a compound of formula (I) may also be used in combination with or include one or more other therapeutic agents, for example other antihistaminic agents for example H4 receptor antagonists, anticholinergic agents, anti-inflammatory agents such as corticosteroids (e.g. fluticasone propionate, beclomethasone dipropionate, mometasone furoate, triamcinolone acetonide, budesonide and fluticasone furoate); or non-steroidal anti-inflammatory drugs (NSAIDs) (e.g. sodium cromoglycate, nedocromil sodium), PDE-4 inhibitors, leukotriene antagonists, lipoxygenase inhibitors, chemokine antagonists (e.g. CCR3, CCR1, CCR2, CCR4, CCR8. CXCR1, CXCR2), IKK antagonists, iNOS inhibitors, tryptase and elastase inhibitors, beta-2 integrin antagonists and adenosine 2a agonists; or beta adrenergic agents (e.g. salmeterol, salbutamol, formoterol, fenoterol, terbutaline, and the beta agonists described in WO 02/66422, WO 02/270490, WO02/076933, WO03/024439 and WO03/072539 and salts thereof); or antiinfective agents e.g. antibiotic agents (such as retapamulin) and antiviral agents.

It will be clear to a person skilled in the art that, where appropriate, the other therapeutic agent(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic agent. It will be clear also that where appropriate, the therapeutic agents may be used in optically pure form.

There is provided, in another aspect, a combination comprising a compound of formula (I) or a pharmaceutically acceptable salt thereof together with one or more (such as one or two, e.g. one) other therapeutically active agents, optionally with one or more pharmaceutically acceptable carriers and/or excipients.

Other histamine receptor antagonists which may be used alone, or in combination with a dual H1/H3 receptor antagonist include antagonists (and/or inverse agonists) of the H4 receptor, for example, the compounds disclosed in Jablonowski et al., J. Med. Chem. 46:3957-3960 (2003).

In another aspect, there is provided a combination comprising a compound of formula (I) or a pharmaceutical acceptable salt thereof and a β₂-adrenoreceptor agonist.

Examples of β₂-adrenoreceptor agonists include salmeterol (which may be a racemate or a single enantiomer, such as the *R*-enantiomer), salbutamol (which may be a racemate or a single enantiomer such as the *R*-enantiomer), formoterol (which may be a racemate or a single diastereomer such as the *R,R*-diastereomer), salrnefamol, fenoterol, carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulfate salt or free base of salbutamol or the fumarate salt of formoterol. In one embodiment, combinations containing a compound of formula (I) may include longer-acting β₂-adrenoreceptor agonists, for example, compounds which provide effective bronchodilation for about 12 h or longer.

Other β₂-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WO01/42193 and WO03/042160.

Examples of β₂-adrenoreceptor agonists include:
3-(4-{[6-({(2*R*)-2-hydroxy-2-[4-hydroxy-3-(hydroxymethyl)phenyl]ethyl}amino)hexyl]oxy} butyl)benzenesulfonamide;
3-(3-{[7-({(2*R*)-2-hydroxy-2-[4-hydroxy-3-hydroxymethyl) phenyl] ethyl}-amino)heptyl]oxy} propyl)benzenesulfonamide;
4-{(1*R*)-2-[(6-{2-[(2,8-dichlorobenryl)oxy]ethoxy}hexyl)amino]-1-hydroxyethyl}-2-(hydroxyl methyl) phenol;
4-{(1*R*)-2-[(6-{4-[3-(cyclopentylsulfonyl)phenyl]butoxy}hexyl)amino]-1-hydroxyethyl}-2-(hydroxylmethyl)phenol;
N-[2-hydroxyl-5-[(1*R*)-1-hydroxy-2-[[2-4-[[(2*R*)-2-hydroxy-2-phenylethyl]amino]phenyl] ethyl]amino]ethyl]phenyl]formamide:
N-2{2-[4-(3-phenyl-4-methoxyphenyl)aminophenyl]ethyl}-2-hydroxy-2-(8-hydroxy-2(1*H*)-quinolinon-5-yl)ethylamine; and
5-[(*R*)-2-(2-{4-[4-(2-amino-2-methyl-propoxy)-phenylamino]-phenyl}-ethylamino)-1-hydroxy -ethyl]-8-hydroxy-1H-quinolin-2-one.

The β-₂adrenoreceptor agonist may be in the form of a salt formed with a pharmaceutically acceptable acid selected from sulfuric, hydrochloric, fumaric, hydroxynaphthoic (for example 1- or 3-hydroxy-2-naphthoic), cinnamic, substituted cinnamic, triphenylacetic, sulfamic, sulfanilic, naphthaleneacrylic, benzoic, 4-methoxybenzoic, 2- or 4-hydroxybenzoic, 4-chlorobenzoic and 4-phenylbenzoic acid.

In another aspect, there is provided a combination comprising a compound of formula (I) or a pharmaceutically acceptable salt thereof and an anti-inflammatory agent.

Anti-inflammatory agents include corticosteroids. Suitable corticosteroids which may be used in combination with the compounds of formula (I) are those oral and inhaled corticosteroids and their pro-drugs which have anti-inflammatory activity. Examples include methyl prednisolone, prednisolone, dexamethasone, fluticasone propionate, 6α,9α-difluoro-11β-hydroxy-16α-methyl-17α [(4-methyl-1,3-thiazole-5-carbonyl)oxy]-3-oxo-androsta-1,4-diene-17β-carbothioic acid *S-*fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate), 6α,9α-difluoro-11β-hydroxy-16α-methyl-3-oxo-17α-propionyloxy-androsta-1,4-diene-17β-carbothioic acid *S*-(2-oxo-tetrahydro-furan-3S-yl) ester, 6α,9α-difluoro-11β-hydroxy-16α-methyl-3-oxo-17α-(2,2,3,3-tetramethycyclo propylcarbonyl)oxy-androsta-1,4-diene-17β-carbothioic acid *S*-cyanomethyl ester and 8α,9α-difluoro-11β-hydroxy-16α-methyl-17α-(1-methycydopropylcarbonyl)oxy-3-oxo-androsta-1,4-diene-17β-carbothioic acid *S*-fluoromethyl ester, beclomethasone esters (for example the 17-propionate ester or the 17,21-dipropionate ester), budesonide, flunisolide, mometasone esters (for example mometasone furoate), triamcinolone acetonide, rofleponide, ciclesonide (16α,17-[[(*R*)-cyclohexylmethylene]bis(oxy)]-11β,21-dihydroxy-pregna-1,4-diene-3,20-dione), butixocort propionate, RPR-106541, and ST-126. Corticosteroids of particular interest may include fluticasone propionate, 6α,9α-difluoro-11β-hydroxy-16α-methyl-17α-[(4-methyl-1,3-thiazole-5-carbonyl)oxy]3-oxo-androsta-1,4-diene-17β-carbothioic acid *S*-fluoromethyl ester, 6α,9α-difluoro-17α[(2-furanylcarbonyl)oxy]-11β-hydroxy-16α-methyl-3-oxo-androsta-1,4-diene-17β-carbothioic acid *S-*fluoromethyl ester, 6α,9α-difluoro-11β-hydroxy-16α-methyl-3-oxo-17α-(2,2,3,3-tetramethycyclopropylcarbonyl)oxy-androsta-1,4-diene-17β-carbothioic acid *S*-cyano methylester, 6α,9α-difluoro-11β-hydroxy-16α-methyl-17α-(1-methycyclopropylcarbonyl) oxy-3-oxo-androsta-1,4-diene-17β-carbothioic acid S-fluoromethyl ester and mometasone furoate. In one embodiment the corticosteroid is 6α,9α-difluore-17α-[(2-furanylcarbonyl)oxy]-11β-hydroxy-16α-methyl-3-oxo-androsta-1,4-diene-17β-carbothioic acid S-fluoromethyl ester (fluticasone furoate) or mometasone furoate.

There is provided, in another aspect, a combination comprising a compound of formula (I) or a pharmaceutically acceptable salt thereof, together with a corticosteroid, such as fluticasone propionate or 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate) or mometasone furoate. Such combinations may be of particular interest for intranasal administration.

In another aspect, there is provided a combination comprising a compound of formula (I) or a pharmaceutically acceptable salt thereof and a glucocorticoid agonist.

Non-steroidal compounds having glucocorticoid agonism that may possess selectivity for transrepression over transactivation and that may be useful in combination therapy include those covered in the following patent application and patents: WO03/082827, WO98154159, WO04/005229, WO04/009017, WO04/018429, WO03/104195, WO03/082787, WO03/082280, WO031059899. WO03/101932, WO02/02565, WO01/16128, WO00/66590, WO03/086294, WO041026248, WO031061651, WO03/08277, WO06/000401, WO06/000398 and WO06/015870.

Anti-inflammatory agents also include non-steroidal anti-inflammatory drugs (NSAID's).

NSAID's include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis (eg. montelukast), iNOS (inducible nitric oxide synthase) inhibitors (e.g. oral iNOS inhibitors), IKK antagonists, tryptase and elastase inhibitors, beta-2 integrin antagonists and adenosine receptor agonists or antagonists (e.g. adenosine 2a agonists), cytokine antagonists (e.g. chemokine antagonists, such as a CCR1, CCR2, CCR3, CCR4, or CCR8 antagonists) or inhibitors of cytokine synthesis, or 5-lipoxygenase inhibitors. iNOS inhibitors include those disclosed in WO93113055, WO98/30537, WO02/50021, WO95/34534 and W099162875.

In another aspect, there is provided a combination comprising a compound of formula (I) or a pharmaceutically acceptable salt thereof and a phosphodiesterase 4 (PDE4) inhibitor. The PDE4-specific inhibitor useful in this embodiment may be any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

Compounds which may be of interest include *cis*-4-cyano-4-(3-cydopentytoxy-4-methoxyphenyl)cyclohexan-1-carboxylic acid, 2-carbomethoxy-4-cyano-4-(3-cyctopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]. Also, *cis*-4-cyano-4-[3-(cyclopentyloxy-4-methoxyphenyl]cyclohexane-1-carboxylic acid (also known as cilomilast) and its salts, esters, prodrugs or physical forms, which is described in U.S. patent 5,552,438 issued 03 September, 1996.

Other PDE4 inhibitors include AWD-12-281 from Elbion (Hofgen, N. et al., 15th EFMC Int. Symp. Med. Chem., (Sept 6-10, Edinburgh) 1998, Abst. P. 98; CAS reference No. 247584020-9); a 9-benzyladenine derivative nominated NCS-613 (INSERM); D-4418 from Chiroscience and Schering-Plough; a benzodiazepine PDE4 inhibitor identified as Cl-1018 (PD-168787) and attributed to Pfizer; a benzodioxole derivative disclosed by Kyowa Hakko in WO99/16765; K-34 from Kyowa Hakko; V-11294A from Napp (Landells, L.J. et a/., Eur. Resp. J. [Ann. Cong. Eur. Resp. Soc. (Sept 19-23, Geneva) 1998] 1998, 12 (Suppl. 28): Abst P2393); roflumilast (CAS reference No 162401-32-3) and a pthalazinone (WO99/47505) from Byk-Gulden; Pumafentrine, (-)-p-[(4aR*,10bS*)-9-ethoxy-1,2,3,4,4a, 10b-hexahydro-8-methoxy-2-methylbenzo[c][1,6]naphthyridin-6-yl]-*N*,*N-*diisopropylbenzamide which is a mixed PDE3/PDE4 inhibitor which has been prepared and published on by Byk-Gulden, now Altana; arofylline under development by Almirall-Prodesfarma; VM554/UM565 from Vemalis; or T-440 (Tanabe Seiyaku; Fuji, K. at al., J. Pharmacol. Exp. Ther., 284(1):162, (1998)), and T2585.

Further compounds which may be of interest are disclosed in the published international patent applications WO04/024728 (Glaxo Group Ltd), WO04/056823 (Glaxo Group Ltd) and WO041103998(Glaxo Group Ltd).

In another aspect, there is provided a combination comprising a compound of formula (I) or a pharmaceutically acceptable salt thereof and an anticholinergic agent.

Anticholinergic agents are those compounds that act as antagonists at the muscarinic receptors, in particular those compounds which are antagonists of the M₁ or M₃ receptors, dual antagonists of the M₁/M₃ or M₂/M₃, receptors or pan-antagonists of the M₁/M₂/M₃ receptors. Exemplary compounds for administration via inhalation include ipratropium (for example, as the bromide, CAS 22254-24-6, sold under the name Atrovent), oxitropium (for example, as the bromide, CAS 30286-75-0) and tiotropium (for example, as the bromide, CAS 136310-93-5, sold under the name Spiriva). Also of interest are revatropate (for example, as the hydrobromide, CAS 262586-79-8) and LAS-34273 which is disclosed in WO01/04118. Exemplary compounds for oral administration include pirenzepine (for example, CAS 28797-61-7), darifenacin (for example, CAS 133099-04-4, or CAS 133099-07-7 for the hydrobromide sold under the name Enablex), oxybutynin (for example, CAS 5633-20-5, sold under the name Ditropan), terodiline (for example, CAS 15793-40-5), tolterodine (for example, CAS 124937-51-5, or CAS 124937-52-6 for the tartrate, sold under the name Detrol), otilonium (for example, as the bromide, CAS 26095-59-0, sold under the name Spasmomen), trospium chloride (for example, CAS 10405-02-4) and solifenacin (for example, CAS 242478-37-1, or CAS 242478-38-2, or the succinate also known as YM-905 and sold under the name Vesicare). Other anticholinergic agents include compounds of formula (XXI), which are disclosed in US patent application 60/487981:

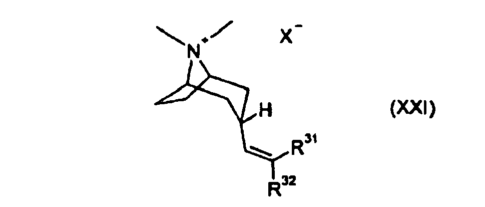

in which a particular orientation of the alkyl chain attached to the tropane ring is endo; R³¹ and R³² are, independently, selected from the group consisting of straight or branched chain lower alkyl groups having e.g. from 1 to 6 carbon atoms, cycloalkyl groups having from 5 to 6 carbon atoms, cycloalkyl-alkyl having 6 to 10 carbon atoms, 2-thienyl, 2-pyridyl, phenyl, phenyl substituted with an alkyl group having not in excess of 4 carbon atoms and phenyl substituted with an alkoxy group having not in excess of 4 carbon atoms;
X' represents an anion associated with the positive charge of the N atom. X- may be, but is not limited to chloride, bromide, iodide, sulfate, benzene sulfonate, and toluene sulfionate, including, for example:
(3-*endo*)-3-(2,2-di-2-thienylethenyl)-8,8-dimethyl-8-azoniabicycio[3.2.1]octane bromide;
(3-*endo*)-3-(2,2-diphenylethenyl)-8,8-dimethyl-8-azoniabicydo[3.2.1]octane bromide;
(3-*endo*)-3-(2,2-diphenylethenyl)-8,8-dimethyl-8-azoniabicydo[3.2.1]octane 4-methyl benzenesulfonate;
(3-*endo*)-8,8-dimethyl-3-[2-phenyl-2-(2-thienyl)ethenyl]-8-azoniabicyclo[3.2.1]octane bromide; and/or
(3-*endo*)-8,8-dimethyl-3-[2-phenyl-2-(2-pyridinyl)ethenyl]-8-azoniabicyclo[3.2.1]octane bromide.

Further anticholinergic agents include compounds of formula (XXII) or (XXIII), which are disclosed in US patent application 601511009: 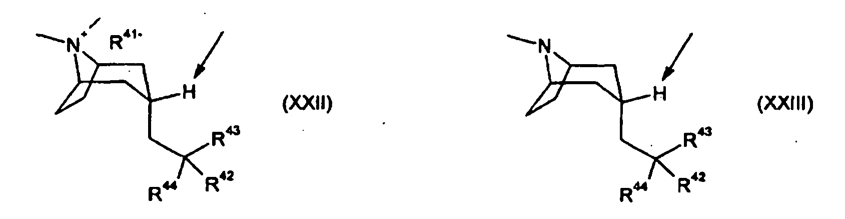 wherein:
the H atom indicated is in the exo position;
R⁴¹⁻ represents an anion associated with the positive charge of the N atom. R1⁻ may be but is not limited to chloride, bromide, iodide, sulfate, benzene sulfonate and toluene sulfonate;
R⁴² and R⁴³ are independently selected from the group consisting of straight or branched chain lower alkyl groups (having for example from 1 to 6 carbon atoms), cycloalkyl groups (having from 5 to 6 carbon atoms), cycloalky-alkyl (having 6 to 10 carbon atoms), heterocycloalkyl (having 5 to 6 carbon atoms) and N or O as the heteroatom, heterocycloalkyl-alkyl (having 6 to 10 carbon atoms) and N or O as the heteroatom, aryl, optionally substituted aryl, heteroaryl, and optionally substituted heteroaryl;
R⁴⁴ is selected from the group consisting of (C₁-C₈)alkyl, (C₃-C₁₂)cycloalkyl, (C₃-C₇)heterocycloalkyl, (C₁-C₆)alkyl(C₃-C₁₂)Cycloalkyl, (C₁-C₆)alkyl(C₃-C₇)heterocycloalkyl, aryl, heteroaryl, (C₁-C₆)alkyl-aryl. (C₁-C₆)alkyl-heteroaryl, -OR⁴⁵, -CH₂OR⁴⁵, -CH₂OH, -CN, -CF₃, -CH₂O(CO)R⁴⁸, -CO₂R⁴⁷, -CH₂NH₂, - CH₂N(R⁴⁷)SO₂R⁴⁵, -SO₂N(R⁴⁷)(R⁴⁸), -CON(R⁴⁷)(R⁴⁸), -CH₂N(R⁴⁸)CO(R⁴⁶). -CH₂N(R⁴⁸)SO₂(R⁴⁶), - CH₂N(R⁴⁸CO₂(R⁴⁵), -CH₂N(R⁴⁸)CONH(R⁴⁷);
R⁴⁵ is selected from the group consisting of (C₁-C₆)alkyl, (C₁-C₆)alkyl(C₃-C₁₂)cycloalkyl, (C₁-C₆)alkyl(C₃-C₇)heterocycloalkyl, (C₁-C₆)alkyl-aryl, (C₁-C₆)alkyl-heteroaryl;
R⁴⁶ is selected from the group consisting of (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocydoalkyl, aryl, heteroaryl, (C₁-C₆)alkyl-aryl, (C₁-C₆)alkyl-heteroaryl;
R⁴⁷ and R⁴⁸ are, independently, selected from the group consisting of H, (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, (C₁-C₆)alkyl-aryl, and (C₁-C₆)alkyl-heteroaryl, including, for example: (Endo)-3-(2-methoxy-2,2-di-thiophen-2-yl-ethyl)-8,8-dimethyl-8-azonia-bicyclo[3.2.1]octane iodide; 3-((Endo)-8-methyl-8-aza-bicyclo[3.2.1]oct-3-yl)-2,2-diphenyl-propionitrile; (Endo)-8-methyl-3-(2,2,2-triphenyl-ethyl)-8-aza-bicyclo[3.2.1]octane; 3-((Endol-8-methyl-8-aza-bicyclo[3.2.1]oct-3-yl)-2,2-diphenyl-propionamide; 3-((Endo)-8-methyl-8-aza-bicyclo[3.2.1]oct-3-yl)-2,2-diphenyl-propionic acid; (Endo)-3-(2-cyano-2,2-diphenyl-ethyl)-8,8-dimethyl-8-azonia-bicyclo[3.2.1]octane iodide; (Endo)-3-(2-cyano-2,2-diphenyl-ethyl)-8,8-dimethyl-8-azonia-bicyclo[3.2.1]octane bromide; 3-((Endo)-8-methyl-8-aza-bicyclo[3.2.1]oct-3-yl)-2,2-diphenyl-propan-1-ol; *N*-Benzyl-3-((endo)-8-methyl-8-aza-bicydo[3.2.1]oct-3-yl)-2,2-diphenyl-propionamide; (Endo)-3-(2-carbamoyl-2,2-diphenyl-ethyl)-8,8-dimethyl-8-azonia-bicyclo[3.2.1]octane iodide; 1-Benzyl-3-[3-((endo)-8-methyl-8-aza-bicyclo[3.2.1]oct-3-yl)-2,2-diphenyl-propyl]-urea; 1-Ethyl-3-[3-((endo)-8-methyl-8-aza-bicyclo[3.2.1]oct 3-yl)-2,2-diphenyl-propyl]-urea; *N*-[3-((Endo)-8-methyl-8-aza-bicydo[3.2.1]oct-3-yl)-2,2-diphenyl-propyl]acetamide; *N*-[3-((Endo)-8-methyl-8-aza-bicyclo[3.2.1]oct-3-yl)-2,2-diphenyl-propyl]-benzamide; 3-((Endo)-8-methyl-8-aza-bicyclo[3.2.1]oct-3-yl)-2,2-di-thiophen-2-yl-propionitrile; (Endo)-3-(2-cyano-2,2-di-thiophen-2-yl-ethyl)-8,8-dimethyl-8-azonia-bicydo[3.2.1]octane iodide; *N*-[3-((Endo)-8-methyl-8-aza-bicyclo[3.2.1]oct-3-yl)-2,2-diphenyl-propyl]-benzene sulfonamide; [3-((Endo)-8-methyl-8-aza-bicyclo[3.2.1]oct-3-yl)-2,2-diphenyl-propyl]-urea; *N*-[3-((Endo)-8-methyl-8-aza-bicyclo[3.2.1]oct-3-yl)-2,2-diphenyl-propyl]-methane sulfonamide; and/or
(Endo)-3-{2,2-diphenyl-3-[(1-phenyl-methanoyl)-amino]-propyl}-8,8-dimethyl-8-azonia-bicyclo[3.2.1]octane bromide.

Particular anticholinergic compounds that may be of use include:
(Endo)-3-(2-methoxy-2,2-di-thiophen-2-yl-ethyl)-8,8-dimethyl-8-azonia-bicyclo[3.2.1] octane iodide;
(Endoy-3-(2-cyano-2,2-diphenyl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane bromide;
(Endo)-3-(2-carbamoyl-2,2-diphenyl-ethyl)-8,8-dimethyl-8-azonia-bicyclo[3.2.1]octane iodide;
(Endo)-3-(2-cyano-2,2-di-thiophen-2-yl-ethyl)-8,8-dimethyl-8-azonia-bicydo[3.2.1]octane iodide; and/or
(Endo)-3-{2,2-diphenyl-3-[(1-phenyl-methanoyl)-amino]-propyl}-8,8-dimethyl-8-azonia-bicyclo[3.2.1]octane bromide.

The combinations referred to above may conveniently be presented for use in the form of a pharmaceutical composition and thus pharmaceutical compositions comprising a combination as defined above optionally together with a pharmaceutically acceptable carriers and/or excipients.

The individual compounds of such combinations may be administered either sequentially in separate pharmaceutical compositions as well as simultaneously in combined pharmaceutical compositions. Additional therapeutically active ingredients may be suspended in the composition together with a compound of formula (I). Appropriate doses of known therapeutic agents will be readily appreciated by those skilled in the art.

The compounds of the invention may be prepared by the methods described below or by similar methods. Thus the following Intermediates and Examples serve to illustrate the preparation of the compounds of the invention, and are not to be considered as limiting the scope of the invention in any way.

### GENERAL EXPERIMENTAL

Throughout the examples, the following abbreviations are used:

| | |
|---|---|
| DCM: | dichloromethane |
| DMF: | *N,N*-dimethylformamide |
| EtOAc: | ethyl acetate |
| DMSO: | dimethylsulfoxide |
| MeOH: | methanol. |
| THF: | tetrahydrofuran |
| MgSO₄: | magnesium sulfate |
| Et₃N: | triethylamine |
| B-Benzyl-9-BBN: | B-Benzyl-9-borabicyclononane |
| LCMS: | Liquid Chromatography Mass Spectrometry |
| MDAP: | Mass-directed Auto-preparative HPLC |
| HPLC: | High Performance Liquid Chromatography |
| RT: | retention time |
| h: | hour(s) |
| min: | minute(s) |

Flash silica gel refers to Merck Art No. 9385; silica gel refers to Merck Art No. 7734.

SCX cartridges are Ion Exchange SPE columns where the stationary phase is polymeric benzene sulfonic acid. These are used to isolate amines.

SCX2 cartridges are Ion Exchange SPE columns where the stationary phase is polymeric propylsulfonic acid. These are used to isolate amines.

Organic solutions were dried either over magnesium or sodium sulfate.

LCMS was conducted on a Supelcosil LCABZ+PLUS column (3.3 cm x 4.6 mm ID) eluting with 0.1% HCO₂H and 0.01 M ammonium acetate in water (solvent A) and 0.05% HCO₂H 5% water in acetonitrile (solvent B), using the following elution gradient 0.0-7min 0%B. 0.7-4.2 min 100%B, 4.2-5.3 min 0%B, 5.3-5.5min 0%8 at a flow rate of 3ml/min. The mass spectra were recorded on a Fisons VG Platform spectrometer using electrospray positive and negative mode (ES+ve and ES-ve).

The Flashmaster II is an automated multi-user flash chromatography system, available, from Argonaut Technologies Ltd, which utilises disposable, normal phase, SPE cartridges (2 g to 100 g).
It provides quaternary on-line solvent mixing to enable gradient methods to be run. Samples are queued using the multi-functional open access software, which manages solvents, flow-rates, gradient profile and collection conditions. The system is equipped with a Knauer variable wavelength uv-detector and two Gilson FC204 fraction-collectors enabling automated peak cutting, collection and tracking.

Mass directed autopreparative HPLC (MDAP) was conducted on a Waters FractionLynx system comprising of a Waters 600 pump with extended pump heads, Waters 2700 autosampler, Waters 996 diode array and Gilson 202 fraction collector on a 10 cm x 2.54 cm internal diameter ABZ+ column, eluting with 0.1 % formic acid in water (solvent A) and 0.1 % formic acid in MeCN (solvent B), using as appropriate elution gradient over 15 min at a flow rate of 20 mlmin⁻¹ and detecting at 200 - 320 nm at room temperature. Mass spectra were recorded on Micromass ZMD mass spectrometer using electro spray positive and negative mode, alternate scans. The software used was MassLynx 3.5 with OpenLynx and FractionLynx options.

Reactions are routinely monitored by methods well known to those skilled in the art, such as TLC, LCMS and/or HPLC. Such methods are used to assess whether a reaction has gone to completion, and reaction times may be varied accordingly.

Intermediates and compounds were named using ACD/Name PRO 6.02 chemical naming software Advanced Chemistry Developments Inc.; Toronto, Ontario, M5H2L3, Canada.

### Intermediates

### Intermediate 1

### 6-Bromo-8-fluoro-2-methylquinoline

Toluene (40 ml) and crotonaldehyde (commercially available, for example, from Aldrich) (4.95 ml) were added to a stirring solution of 4-bromo-2-fluoroaniline (commercially available, for example, from Aldrich) (5.7 g) in 5M hydrochloric acid (135 ml) and the resulting mixture was heated at 100 °C for 3 h. The mixture was allowed to cool and the layers were separated. The aqueous phase was basified with 5M sodium hydroxide solution causing a purple solid to precipitate. This solid was collected by filtration, washed with water, dissolved in DCM and loaded onto a 100 g silica Flashmaster II cartridge. The cartridge was eluted with a 0-100% EtOAc in cyclohexane gradient over 60 min. Evaporation of the solvent from appropriate fractions gave the *title compound* (3.64 g). LCMS RT= 2.96 min, ES+ve *m*/*z* 240/242 [M+H]⁺.

The solvent was evaporated from a further set of fractions giving a residue that was purified by Flashmaster II on a 50 g silica cartridge using a 0-50% EtOAc in cyclohexane gradient over 40 min. Evaporation of the solvent from appropriate fractions gave the *title compound* (428 mg). LCMS RT = 2.96 min, ES+ve *m*/*z* 240/242 [M+H]⁺.

### Intermediate 2

### 6-Bromo-2-methyl-8-(1-piperazinyl)quinoline

A mixture of 6-bromo-e-fluoro-2-methylquinoline (for example, as prepared for Intermediate 1) (1.43 g) and piperazine (commercially available, for example, from Aldrich) (5.134 g) was heated at 150 °C by microwave irradiation in a Smith Creator microwave oven for 30 min. The cooled mixture was partitioned between DCM (50 ml) and water (50 ml) and the organic phase was dried over sodium sulphate. Evaporation of the solvent gave the *title compound* (2.055 g). LCMS RT = 2.08 min, ES+ve *m*/*z* 306/308 [M+H]⁺.

### Intermediate 3

### 1,1-Dimethylethyl 4-(6-bromo-2-methyl-8-qulnolinyl)-1-piperazinecarboxylate

To a stirring mixture of 6-bromo-2-methyl-8-(1-piperazinyl)quinoline (for example, as prepared for Intermediate 2) (2 g), 4-dimethylaminopyridine (commercially available, for example, from Aldrich) (80 mg) and Et₃N (2.73 ml) in acetonitrile (26 ml) at 0 °C, was added, a solution of di-*tert*-butyl dicarbonate (2.14 g) in acetonitrile (4 ml). The mixture was stirred at 0 °C for 15 min, then at room temperature overnight. The solvent was evaporated and the residue was partitioned between DCM (100 ml) and water (100 ml). The organic layer was dried over MgSO₄. and the solvent was evaporated. The residue was purified by Flashmaster II on a 100 g silica cartridge using 0-100% EtOAc in DCM over 40 min. Evaporation of the solvent from appropriate fractions gave the title *compound* (2.1708 g). LCMS RT = 3.78 min, ES+ve *m*/*z* 406/408 [M+H]⁺.

### Intermediate 4

### 1,1-Dimethylethyl 4-(6-bromo-2-formyl-8-quinolinyl)-1-piperazinecarboxylate

To a stirring solution of selenium dioxide (494 mg) in 1,4-dioxane (20 ml) at 55 °C under an atmosphere of nitrogen, was added dropwise over 2 h, a solution of 1,1-dimethylethyl 4-(6-bromo-2-methyl-8-quinolinyl)-1-piperazinecarboxylate (for example, as prepared for Intermediate 3) (1 g) in 1,4-dioxane (7.5 ml). The resulting mixture was stirred at 80 °C overnight, filtered and evaporated giving a residue that was dissolved in DCM and loaded onto a 20 g silica SPE cartridge. The cartridge was eluted with DCM and the solvent was evaporated from appropriate fractions to give the *title compound* (1.02 g). LCMS RT = 3.74 min, ES+ve *m*/*z* 420/422 [M+H]⁺.

### Intermediate 5

### 1,1-Dimethylethyl 4-{6-bromo-2-[(1E-3-(methyloxy)-3-oxo-1-propen-1-yl]-8-quinolinyl}-1-piperazinecarboxylate

A mixture of 1,1-dimethylethyl 4-(6-bromo-2-formyl-8-quinolinyl)-1-piperazinecarboxylate (for example, as prepared for Intermediate 4) (1 g) and methyl (triphenylphosphoranylidene)acetate (commercially available, for example, from Aldrich) (875 mg) in anhydrous THF (15 ml) was stirred at 65 °C under a nitrogen atmosphere for 18 h: A further quantity of ylid (850 mg) was added and the mixture heated for a further 22 h. The reaction mixture was diluted with toluene (20 ml) and loaded onto a 20 g silica SPE cartridge. The cartridge was eluted with toluene and the solvent was evaporated from appropriate fractions. The residue was further purified by Flashmaster II on a 70 g silica cartridge using a 0-100% EtOAc in cyclohexane gradient. Evaporation of the solvent from appropriate fractions gave the *title compound* (802 mg). LCMS RT = 3.95 min, ES+ve *m*/*z* 476/478 [M+H]⁺.

### Intermediate 6

### 1,1-Dimethylethyl 4-[2-[(1E)-3-(methyloxy)-3-oxo-1-propen-1-yl]-6-(phenylmethyl)-8-quinolinyl]-1-piperazinecarboxylate

A mixture of 1,1-dimethylethyl 4-{6-bromo-2-[(1*E*)-3-(methyloxy)-3-oxo-1-propen-1-yl]-8-quinolinyl} 1-piperazinecarboxylate (for example, as prepared for Intermediate 5) (200 mg), potassium carbonate (116.1 mg) and [1,1'-bis(diphenylphosphino)ferrocene)palladium (II) chloride (15.4 mg, 5 mol%) in DMF (1.3 ml) was treated with *B*-Benzy)-9-BBN (1.68 ml of 0.5M solution in THF). The mixture was heated at 65 °C in a sealed Reactivial™ for 3 h. Further quantities of [1,1'-bis(diphenylphosphino)ferrocene)]palladium (II) chloride (15 mg) and *B*-Benzyl-9BBN (1.68 ml of 0.5M solution in THF) were added and the mixture stirred for a further 92 h. The solvent was evaporated from the reaction mixture giving the *title compound* that was used without further purification. LCMS RT = 4.01 min, ES+ve *m*/*z* 488 (M+H)⁺.

### Intermediate 7

### Methyl (2E)-3-[6-(phenylmethyl)-8-(1-piperazinyl)-2-quinolinyl]-2-propenoate

A solution of 1,1-dimethylethyl 4-[2-(1*E*)-3-(methyloxy)-3-oxo-1-propen-1-yl]-6-(phenylmethyl)-8-quinolinyl]-1-piperazinecarboxylate (for example, as prepared for Intermediate 6) in DCM (10 ml) was treated with trifluoroacetic acid (3 ml) and the resulting mixture was stirred at room temperature under a nitrogen atmosphere for 20 min. The mixture was evaporated giving a residue that was treated with toluene and re-evaporated. The residue was dissolved in a minimum of MeOH and loaded onto a MeOH-conditioned SCX ion-exchange cartridge (20 g). The cartridge was eluted with MeOH (3 column volumes) and then 2M ammonia in MeOH (3 column volumes). Evaporation of the solvent from the ammonia-containing fractions gave a crude sample of the *title compound* that was used without further purification. LCMS RT = 2.65 min, ES+ve *m*/*z* 388 [M+H]⁺.

### Intermediate 8

### Methyl 3-[6-(phenylmethyl)-8-(1-piperazinyl)-2-quinolinyl]propanoate

A mixture of methyl (2*E*)-3-[6-(phenylmethyl)-8-(1-piperazlnyl)-2-quinolinyl]-2-propenoate (for example, as prepared for Intermediate 7), acetic acid (1 ml) and 10% palladium on charcoal (200 mg) in EtOAc (25 ml) was hydrogenated at atmospheric pressure for 2 h. The reaction mixture was filtered through celite and the solvent was evaporated. The residue was purified by Biotage Flash Chromatography on a 12M KP-Sil cartridge eluting with 5% (2M ammonia in MeOH) in DCM. Evaporation of the solvent from appropriate fractions gave a crude sample of the *title compound* (55 mg). LCMS RT = 2.57 min, ES+ve *m*/*z* 390 [M+H]⁺.

### Intermediate 9

### Methyl 3-[8-{4-[2-({[3-(hexahydro-1H-azepin-1-yl)propyl]oxy}phenyl)ethyl]-1-piperazinyl}-6-(phonylmethyl)-2-quinolinyl]propanoate

A mixture of methyl 3-[6-(phenylmethyl)-8-(1-piperazinyl)-2-quinolinyl]propanoate (for example, as prepared for Intermediate 8) (55 mg), sodium bicarbonate (25 mg) and 2-(4-{[3-(hexahydro-1*H* azepin-1-yl)propyl]oxy}phenyl)ethyl methanesulfonate (for example, as prepared for Intermediate 15) (50 mg) in acetonitrile (1.5 ml) was heated at 80 °C under a nitrogen atmosphere for 65 h. The reaction mixture was filtered and evaporated. The residue was dissolved in MeOH - DMSO (1:1, 2 ml) and purified by MDAP. Evaporation of the solvent from appropriate fractions gave the *title compound* (33 mg). LCMS RT = 2.51 min, ES+ve *m*/*z* 649 [M+H]⁺.

### Intermediate 10

### 1,1-Dimethylethyl 4-{6-ethyl-2-[(1E)-3-(methyloxy)-3-oxo-1-propen-1-yl]-8-quinolinyl}-1-piperazinecarboxylate

A mixture of 1,1-dimethylethyl 4-{6-bromo-2-[(1*E*)-3-(methyloxy)-3-oxo-1-propen-1-yl}-8-quinolinyl)-1-piperazinecarboxylate (for example, as prepared for Intermediate 5) (200 mg), potassium carbonate (116.1 mg) and [1,1'-bis(diphenylphosphino)ferrocene)palladium (II) chloride (15.4 mg, 5 mol%) in DMF (1.3 ml) was treated with triethylborane (0.84 ml of 1.0M solution in THF). The mixture was heated at 65 °C in a sealed Reactivial™ for 3 h. Further quantities of [1,1'-bis(diphenylphosphino)ferrocene)]palladium (II) chloride (15 mg) and triethylborane (0.84 ml of 1.0M solution in THF) were added and the mixture stirred for a further 92 h. The solvent was evaporated from the reaction mixture and the residue was dissolved in DCM and loaded onto a silica Flashmaster cartridge (20 g). The cartridge was eluted with a 0-100% EtOAc in cyclohexane gradient over 40 min. Appropriate fractions were combined and the solvent was evaporated to give the *title compound* (98 mg). LCMS RT = 3.91 min, ES+ve *m*/*z* 426 [M+H]⁺.

### Intermediate 11

### Methyl 3-[6-ethyl-8-(1-piperazinyl)-2-quinolinyl]propanoate

A solution of 1,1-dimethylethyl 4-{8-ethyl-2-[(1*E*)-3-(methyloxy)-3-oxo-1-propen-1-yl}-8-quinolinyl}-1-piperazinecarboxylate (for example, as prepared for Intermediate 10) (97 mg) in DCM (10 ml) was treated with trifluoroacetic acid (2 ml) and the resulting mixture was stirred at room temperature under a nitrogen atmosphere for 1 h. The mixture was evaporated giving a residue that was treated with toluene and re-evaporated. The residue was dissolved in a mixture of EtOAc (20 mn and acetic acid (1 ml) and hydrogenated at atmospheric pressure in the presence of 10% palladium on charcoal (100 mg) for 1 h. The reaction mixture was filtered through a celite cartridge and the filtrate was evaporated. The residue was dissolved in MeOH and loaded onto a MeOH-conditioned SCX ion-exchange cartridge (10 g). The cartridge was eluted with MeOH and then 2M ammonia in MeOH. Evaporation of the solvent from the ammonia-containing fractions gave the *title compound* (73 mg). LCMS RT = 2.22 min, ES+ve *m*/*z* 328 [M+H]⁺.

### Intermediate 12

### Methyl 3-(6-ethyl-8-{4-[2-(4-{[3-(hexahydro-1H-azepin-1-yl)propyl]oxy}phenyl)ethyl]-1-piperazinyl}-2-quinolinyl)propanoate

A mixture of methyl 3-[6-(ethyl)-8-(1-piperazinyl)-2-quinolinyl]propanoate (for example, as prepared for Intermediate 11) (72.9 mg), sodium bicarbonate (50 mg) and 2-(4-{[3-(hexahydro-1*H*-azepin-1-yl)propyl]oxy}phenyl)ethyl methanesulfonate (for example, as prepared for Intermediate 15) (80 mg) in acetonitrile (2 ml) was heated with stirring at 80 °C for 20 h. The reaction mixture was diluted with DCM (10 ml) and MP-isocyanate resin (200 mg, loading = 1.58 mmol/g) was added. The mixture was shaken for 4 h, filtered and evaporated to give a crude sample of the *title compound* that was used without further purification.

### Intermediate 13

### 2-{4-[(3-Chloropropyl)oxy]phenyl}ethanol

4-(2-Hydroxyethyl)phenol) (commercially available, for example from Aldrich) (10 g, 72 mmol) was dissolved in 2-butanone (250 ml) then potassium carbonate (19.9 g, 0.144 mol) was added then 1-bromo-3-chloropropane (commercially available, for example, from Aldrich) (8.54 ml, 0.144 mol) was added and the reaction mixture heated at 80 °C for 18 h. The cooled reaction mixture was diluted with water (500 ml), layers separated and aqueous extracted with DCM (2 x 200 ml). The combined organic extracts were dried (MgSO₄), evaporated *in vacuo* and purified by Flashmaster (3 x 100 g silica cartridges) eluted with 0-100% EtOAc-cyclohexane over 40 min to give *the title compound* (14.12 g). LCMS RT = 2.84 min ES+ve *m*/*z* 232 (M+NH₄)⁺

### Intermediate 14

### 2-(4-{[3-(Hexahydro-1H-azepin-1-yl)propyl]oxy}phenyl)ethanol

A solution of 2-{4-[(3-chtoropropyl)oxy]phenyl}ethanol (for example, as prepared for Intermediate 13) (14 g, 0.065 mol) in 2-butanone (200 ml) was treated with potassium carbonate (17.96 g, 0.13 mol), potassium iodide (1.24 g, 7.5 mmol), hexahydro-1*H*-azepine (commercially available, for example, from Aldrich) (14.71 ml, 0.1308 mol) and heated at 80 °C under nitrogen for 18 h. The cooled reaction mixture was diluted with water (300 ml), layers separated and the aqueous extracted with DCM (2 x 200 ml). The combined organic extracts were dried (MgSO₄) and evaporated *in* vacuo to give a yellow oil (23 g). A portion of this (10 g) was purified by Flashmaster (100 g silica cartridge), eluted with 0-100% EtOAc-cyclohexane over 15 min, then 100% EtOAc for 10 min, then 0-10% (10% aq ammonia-MeOH)-DCM for 15 min, then 10% of (10% aq ammonia-MeOH) - DCM for 10 min to give *the title compound* (5.3 g). LCMS RT =1.9 min, ES+ve *m*/*z* 278 (M+H)⁺.

### Intermediate 15

### 2-(4-{[3-(Hexahydro-1H-azepin-1-yl)propyl]oxy}phenyl)ethyl methanesulfonate

A solution of 2-(4-{[3-(hexahydro-1*H*-azepin-1-yl)propyl]oxy}phenyl)ethanol (for example, as prepared for Intermediate 14) (80 mg, 0.29 mmol) in DCM (2 ml) and diisopropylethylamine (0.06 ml, 0.34 mmol) was treated with methanesulfonyl chloride (0.026 ml, 0.34 mmol) at 20 °C and the mixture was stirred for 2 h. The solution was diluted with DCM (10 ml) and saturated sodium bicarbonate solution (10 ml), and the phases separated using a hydrophobic frit. The organic phase was concentrated under reduced pressure to give *the title compound* (0.101 g, 100%). LCMS RT = 2.18 min, ES+ve *m*/*z* 356 (M+H)⁺.

### Intermediate 16

### 1,1-Dimethylethyl 4-[2-(4-{[(1,1-dimethylethyl)(dimethyl)silyl]oxy}phenyl)ethyl]-1-piperazinecarboxylate

A solution of *N*-*tert*-butoxycarbonylpiperazine (commercially available, for example, from Lancaster) (75.74 g, 407 mmol) in acetonitrile (500 ml) was treated with sodium bicarbonate (45.57 g, 542 mmol) and the mixture was stirred for 15 min. A solution of 2-(4-{[(1,1-dimethylethyl)(dimethyl)silyl]oxy}phenyl)ethylmethanesulfonate (disclosed in W02003091204; see page 61, Example 28 part (ii)) (103.32 g, 313 mmol) in acetonitrile (600 ml) was added to the mixture over 20 min and heated to reflux under nitrogen for 23 h. HPLC indicated the presence of 9% starting material and the mixture was heated to reflux for a further 19 h. The mixture was allowed to cool to room temperature and the acetonitrile was removed under reduced pressure. The residue was partitioned between EtOAc (500 ml) and water (500 ml). The aqueous phase was extracted with EtOAc (100 ml) and the combined organic solutions were washed with brine (4 x 50 ml), dried (MgSO₄) and evaporated. The residue was purified by chromatography on silica eluting with EtOAc-cyclohexane (1:4 to 1: 1) to give the *title compound* (101.7 g) NMR δ (CDCl₃) 7.05 (2H, d). 6.76 (2H, d), 3.48-3.44 (4H, m), 2.76-2.71 (2H, m), 2.60-2.54 (2H, m), 2.49-2.43 (4H, m), 1.47 (9H, s), 0.98 (9H, s), 0.19 (6H, s) and a less pure batch (5.5 g).

### Intermediate 17

### 1,1-Dimethylethyl 4-[2-(4-hydroxyphenyl)ethyl]-1-piperazinecarboxylate

A solution of 1.1-dimethylethyl 4-[2-(4-{[(1,1-dimethylethyl)(dimethyl)silyl]oxy} phenyl)ethyl]-1-piperazinecarboxylate (for example, as prepared for Intermediate 16) (101.7 g, 242 mmol) in THF (1 L) was treated with tetrabutylammonium fluoride solution in THF (1M, 266 ml) and the mixture was stirred at room temperature for 3 h under nitrogen. The solvent was removed under reduced pressure and the residue was partitioned between water (300 ml) and EtOAc (300 ml). Crystallisation occurred in the separating funnel and the crystals were collected by filtration and washed with EtOAc to give *the title compound* (29.16 g). The filtrate was partitioned between water and EtOAc. The aqueous was re-extracted with EtOAc and the combined organic extracts were washed with brine, dried (MgSO₄) and evaporated. The residue was diluted with diethyl ether and the resulting solid was collected by filtration to give *the title compound* (36.39 g) ¹H NMR 6 (CDCl₃) 7.05 (2H, d, *J* 8 Hz), 6.75 (2H, d, *J* 8 Hz) 5.08 (1H, br), 3.48 (4H, m), 2.77-2.71 (2H, m), 2.61-2.55 (2H, m), 2.50-2.45 (4H, m), 1.47 (9H, s).

### Intermediate 18

### 1,1-Dimethylethyl 4-(2-{4-[(3-chloropropyl)oxy]phenyl}ethyl)-1-piperazinecarboxylate

A solution of 1,1-dimethylethyl 4-[2-(4-hydroxyphenyl)ethyl]-1-piperazinecarboxylate (for example, as prepared for Intermediate 17) (36.39 g, 119 mmol) in methyl ethyl ketone (300 ml) was treated with potassium carbonate (18.05 g, 131 mmol) and the mixture was stirred for 1 h at room temperature. 1-Bromo-3-chloropropane (commercially available, for example, from Aldrich) (18.1 ml, 183 mmol) was added and the mixture was stirred at room temperature under nitrogen overnight and then heated to reflux for a further 24 h. The mixture was allowed to cool to room temperature, diluted with EtOAc (200 ml), washed four times with sodium hydroxide solution (2M, 50 ml) and then brine. The solution was dried over MgSO₄ and evaporated. The residue (49.12 g) was purified by column chromatography on silica eluting with EtOAc-cyclohexane (1:1) to give *the title compound* (25.78 g) ¹H NMR δ (CDCl₃) 7.12 (2H, d, *J* 8 Hz), 6.84 (2H, d, *J* 8 Hz), 4.10 (2H, t, *J* 6 Hz), 3.75 (2H, t, *J* 6 Hz), 3.47 (4H, m), 2.78-2.71 (2H, m), 2.60-2.54 (2H, m), 2.46 (4H, m), 2.23 (2H, m), 1.47 (9H, s).

### Intermediate 19

### 1,1-Dimethylethyl 4-[2-(4-{[3-(hexahydro-1H-azepin-1-yl)propyl]oxy}phenyl) ethyl]-1-piperazinecarboxylate

A mixture of 1,1-dimethylethyl 4-(2-{4-[(3-chloropropyl)oxy]phenyl}ethyl)-1-piperazinecarboxylate (for example, as prepared for Intermediate 18) (11.5 g, 30 mmol), homopiperazine (commercially available, for example, from Aldrich) (5.07 ml, 45 mmol), potassium iodide (0.1 g), potassium carbonate (8.3 g, 60 mmol) in 2-butanone (100 ml) was heated to reflux for 16 h and then cooled to room temperature. The reaction mixture was partitioned between DCM and water. The organic phase was dried (hydrophobic frit) and concentrated. The residue was purified by chromatography on a biotage cartridge eluting with EtOAc, then a gradient of 0 to 10% MeOH (containing 1% Et₃N) in DCM to give *the title compound* (11.6 g) LCMS RT = 1.92 min, ES+ve *m*/*z* 446 (M+H)⁺.

### Intermediate 20

### 1-[3-({4-[2-(1-Piperazinyl)ethyl]phenyl}oxy)propyl]hexahydro-1H-azepine

A solution of 1,1-dimethylethyl 4-[2-(4-{[3-(hexahydro-1*H*-azepin-1-yl)propyl]oxy}phenyl)ethyl]-1-piperazinecarboxylate (for example, as prepared for Intermediate 19) (11.6 g, 26.06 mmol) in DCM (100 ml) was treated with trifluoroacetic acid (20 ml) and the mixture was stirred for 3 h. Additional trifluoroacetic acid (30 ml) was added and the reaction mixture was stirred for a further 2 h. The reaction mixture was concentrated *in vacuo* and then applied to a SCX cartridge, washed with MeOH and then eluted with 2M ammonia in MeOH. The basic fractions were concentrated under reduced pressure to give *the title compound* (9 g). LCMS RT = 1.1 min, *m*/*z* 346 (M+H)⁺.

### Intermediate 21

### Ethyl (2E)-3-(8-hydroxy-2-quinolinyl)-2-propenoate

A solution of (ethoxycarbonylmethyl)triphenylphosphonium bromide (commercially available, for example, from Aldrich) (5.3 g, 12.3 mmol) in anhydrous THF (50 ml) was cooled to 0 °C under nitrogen and then sodium hydride (60% oil dispersion, 0.51 g, 12.7 mmol) was added and the mixture was stirred for 30 min. A solution of 8-hydroxyquinoline-2-carboxaldehyde (commercially available, for example, from Acros) (2.0 g, 11.5 mmol) in THF (50 ml) was added dropwise to the above solution and the mixture was stirred for 1 h. The mixture was diluted with water and the aqueous layer was extracted with DCM (3x). The combined organic layers were washed with brine, dried (MgSO₄) and evaporated. The residue was purified by chromatography (Flashmaster) eluting with 0 to 20% MeOH in DCM to give *the title compound* (2.19 g) LCMS RT = 2.82 min and 3.19 min, (1:4, Z and *E* isomers), ES+ve *m*/*z* 244 (M+H)⁺.

### Intermediate 22

### Ethyl (2E)-3-(8-{[(trifluoromethyl)sulfonyl]oxy}-2-quinolinyl)-2-propenoate

A solution of ethyl (2*E*/*Z*)-3-(8-hydroxy-2-quinolinyl)-2-propenoate (for example, as prepared for Intermediate 21) (1 g, 4.11 mmol) in anhydrous pyridine (5 ml) was cooled to 0 °C under nitrogen. Triflic anhydride (1.04 ml, 6.18 mmol) was added and the mixture was stirred at 0 °C for 45 min. The reaction mixture was allowed to warm up to room temperature, stirred overnight, and then poured onto ice. The mixture was extracted with DCM (3x) and the combined organic layers were washed with brine, dried (MgSO₄) and evaporated. The residue was purified by column chromatography on silica gel eluting with 0 to 3% EtOAc in DCM to give *the title compound* (1.86 g) LCMS RT = 3.57 min ES+ve *m*/*z* 376 (M+H)⁺.

### Intermediate 23

### Methyl (2E)-3-(8-{4-[2-(4-{[3-(hexahydro-1H-azepin-1-yl)propyl]oxyl}phenyl)ethyl]-1-piperazinyl}-2-quinolinyl)-2-propenoate and ethyl (2E)-3-(8-{4-[2-(4-{[3-(hexahydro-1H-azepin-1-yl)propyl]oxy}phenyl)ethyl]-1-piperazinyl}-2-quinolinyl)-2-propenoate

A mixture of 1-[3-({4-[2-(1-piperazinyl)ethyl]phenyl}oxy)propyl]hexahydro-1*H*-azepine (for example, as prepared for Intermediate 20) (1.68 g, 4.87 mmol), caesium carbonate (4.33 g, 13.3 mmol), tris(dibenzylideneacetone)dipalladium (0) (39 mg, 0.097 mmol) and 2-(dicyclohexylphosphino)-2'-(*N*,*N*-dimethylamino)biphenyl (77 mg, 0.195 mmol) was treated with a solution of ethyl (2*E*)-3-(8-{[(trifluoromethyl)sulfonyl]oxy}-2-quinolinyl)-2-propenoate (for example, as prepared for Intermediate 22) (1.66 g, 4.43 mmol) in anhydrous THF (25 ml) and the mixture was heated to reflux overnight under nitrogen. The solvent was removed under reduced pressure and the residue was purified by column chromatography on silica gel eluting with EtOAc, followed by 0 to 10% MeOH (containing 1% Et₃N)-DCM to give a mixture of *the title compounds* (1.68 g). LCMS RT = 2.13 min ES+ve *m*/*z* 557 (M+H)⁺, and RT = 2.22 min ES+ve *m*/*z* 571 (M+H)⁺.

### Intermediate 24

### Methyl 3-(8-{4-[2-(4-{[3-(hexahydro-1H-azepin-1-yl)propyl]oxy}phenyl)ethyl]-1-piperazinyl}-2-quinolinyl)propanoate and ethyl 3-(8-{4-[2-(4-{[3-(hexahydro-1H-azepin-1-yl)propyl]oxy}phenyl)ethyl]-1-piperazinyl}-2-quinolinyl)propanoate

A solution of a mixture of methyl (2*E*)-3-(8-{4-[2-(4-{[3-(hexahydro-1*H*-azepin-1-yl)propyl]oxy}phenyl)ethyl]-1-piperazinyl}-2-quinolinyl)-2-propenoate and ethyl (2*E*)-3-(8-{4-[2-(4-{[3-(hexahydro-1*H*-azepin-1-yl)propyl]oxy}phenyl)ethyl]-1-piperazinyl}-2-quinolinyl)-2-propenoate (for example, as prepared for Intermediate 23) (400 mg, 0.72 mmol) in EtOAc (15 ml) was hydrogenated over 10% palladium on carbon (80 mg) overnight. The catalyst was removed by filtration and the filtrate was evaporated to give *the title compounds* (370 mg) LCMS RT = 2.07 min ES+ve *m*/*z* 559 (M+H)⁺, and RT = 2.16 min ES+ve *m*/*z* 573 (M+H)⁺.

### Intermediate 26

### Methyl (2E)-3-(8-nydroxy-2-quinolinyl)-2-propenoate and methyl (2Z)-3-(8-hydroxy-2-quinolinyl)-2-propenoate

A solution of 8-hydroxy-2-quinolinecarbaldehyde (commercially available, for example, from Fluka and/or Acros) (3.81 g) in anhydrous toluene (40 ml) was treated portionwise with (carbomethoxymethylene) triphenylphosphorane (commercially available, for example, from Aldrich and/or Alfa Alsar) (7.36 g) and the resulting mixture was heated at 50 °C for 1 h. The solvent was evaporated and the residue was dissolved in MeOH and purified on a 70 g SCX-2 ion-exchange cartridge to give a mixture of the *title compounds* (4.98 g). LCMS RT= 3.09 min, ES+ve *m*/*z* 230 [M+H]⁺ and RT= 2.64 min, ES+ve *m*/*z* 230 [M+H]⁺.

### Intermediate 26

### Methyl 3-(8-hydroxy-2-quinolinyl)propanoate

A solution of a mixture of methyl (2*E*)-3-(8-hydroxy-2-quinolinyl)-2-propenoate and methyl (2Z)-3-(8-hydroxy-2-quinolinyl)-2-propenoate (for example, as prepared for Intermediate 25) (4.98 g) in EtOAc (100 ml) was hydrogenated in the presence of 10% w/w palladium on carbon (3.8 g) for 1 h. The reaction mixture was filtered through celite and the solvent was evaporated to give the *title compound* (4.72 g). LCMS RT= 2.40 min, ES+ve *m*/*z* 232 [M+H]⁺.

### Intermediate 27

### Methyl 3-(8-{[(trifluoromethyl)sulfonyl]oxy}-2-quinolinyl)propanoate

A solution of methyl 3-(8-hydroxy-2-quinolinyl)propanoate (for example, as prepared for Intermediate 26) (4.72 g) in DMF (50 ml) was treated with N-phenyl bis(trifluoromethanesulfonimide) (commercially available, for example, from Aldrich) (8.75 g) and Et₃N (3 ml). The resulting mixture was stirred at room temperature for 24 h. Additional quantities of N-phenyl, bis(trifluoromethanesulfonimide) (3.65 g) and Et₃N (1.42 ml) were added and the mixture was stirred for a further 6 h. The reaction mixture was partitioned between water and toluene and the organic phase was washed with saturated brine, dried over anhydrous sodium sulphate and evaporated. A 4 g sample of the resulting residue was purified by Flashmaster II on a 100 g silica cartridge using a 0-100% gradient of DCM - cyclohexane. The solvent was evaporated from appropriate fractions to give the *title compound* (1.76 g). LCMS RT= 3.44 min, ES+ve *m*/*z* 364 [M+H]⁺. A further 5.5 g sample of the crude material was purified by Flashmaster II on a 100 g silica cartridge using a 0-100% EtOAc - cyclohexane gradient. The solvent was evaporated from appropriate fractions to give the *title compound* (1.70 g). LCMS RT= 3.43 min, ES+ve *m*/*z* 364 [M+H]⁺. Impure fractions from both of the above purifications were combined and re-purfied by Flashmaster II on a 100 g silica cartridge using a 0-50% EtOAc in cyclohexane gradient. The solvent was evaporated from appropriate fractions to give the *title compound* (2.67 g). LCMS RT= 3.43 min, ES+ve *m*/*z* 364 [M+H]⁺.

### Intermediate 28

### 1,1-Dimethylethyl 4-{2-[3-(methyloxy)-3-oxopropyl]-8-quinolinyl}-1-piperazinecarboxylate

A mixture of methyl 3-(8-{[(trifluoromethyl)sulfonyl]oxy}2-quinolinyl)propanoate (for example, as prepared for Intermediate 27) (417 mg), 1,1-dimethylethyl 1-piperazinecarboxylate (commercially available, for example from Aldrich) (235 mg), caesium carbonate (1.124 g), tris(dibenzylideneacetone)dipalladium(0) (23 mg) and [2-(dicyclohexylphosphino)-2'-(*N*,*N-*dimethylamino)]biphenyl (20 mg) in THF (6 ml) was heated overnight at 80 °C in a Reacti-Vial^{™}.
The reaction was quenched by the addition of 2M hydrochloric acid and then neutralised with sodium bicarbonate solution. The mixture was extracted with DCM and the extracts were washed with saturated brine, dried over anhydrous sodium sulphate and evaporated to give the *title compound* (510 mg). LCMS RT= 3.36 min, ES+ve *m*/*z* 400 [M+H]⁺.

### Intermediate 29

### Methyl 3-[8-(1-piperazinyl)-2-quinolinyl]propanoate

A solution of 1,1-dimethylethyl 4-{2-[3-(methyloxy)-3-oxopropyl]-8-quinolinyl}-1-piperazinecarboxylate (for example, as prepared for Intermediate 28) (510 mg) in DCM (30 ml) was treated with trifluoroacetic acid (5 ml) and the mixture was stirred at room temperature for 30 min.
The solvent was evaporated giving a residue that was dissolved in MeOH and purified on a 10 g SCX ion-exchange cartridge to give the *title compound* (330 mg). LCMS RT= 1.99 min, ES+ve *m*/*z* 300 [M+H]⁺.

### Intermediate 30

### Methyl 3-(8-{4-[2-(4-{[3-(hexahydro-1H-azepin-1-yl)propyl]oxy}phenyl)ethyl-1-piperazinyl}-2-quinolinyl)propanoate

Methyl 3-[8-(1-piperazinyl)-2-quinolinyl]propanoate (for example, as prepared for Intermediate 29) (93 mg) and sodium bicarbonate (52 mg) were added to a solution of 2-(4-{[3-(hexahydro-1*H-*azepin-1-yl)propyl]oxy}phenyl)ethyl methanesulfonate (for example, as prepared for Intermediate 15) (111 mg) in acetonitrile (2 ml) and the resulting mixture was stirred at 80 °C overnight. The mixture was filtered (Varian Bond Elut Reservoir) and purified by MDAP to give the *title compound* (34 mg).
LCMS RT= 2.20 min, ES+ve *m*/*z* 559 [M+H]⁺.

### Intermediate 31

### 1,1-Dimethylethyl 4-{6-bromo-2-[3-(methyloxy)-3-oxopropyl]-8-quinolinyl}-1-piperazinecarboxylate

To a stirred solution of 1,1-dimethylethyl 4-{6-bromo-2-[(1*E*)-3-(methyloxy)-3-oxo-1-propen-1-yl]-8-quinolinyl}-1-piperazinecarboxylate (for example as prepared for Intermediate 5) (2.38 g) in THF (50 ml) was added water (1.8 ml), followed by hydrido(triphenylphosphine)copper (I) hexamer (2.35 g). The mixture was stirred at room temperature for 45 min, then a further amount of hydrido(triphenylphosphine)copper (1) hexamer (1.18 g) was added and stirring continued for 1 h.
The mixture was filtered through a pad of celite and the filtrate was evaporated. The residue was purified by Flashmaster 11 on a 100 g silica cartridge eluting with a 0-50% EtOAc in cyclohexane gradient over 40 min. Evaporation of the solvent from appropriate fractions gave *the title compound* (2.29 g). LCMS RT = 3.75 min, ES+ve *m*/*z* 478/480 [M+H]⁺.

### intermediate 32

### 1,1-Dimethylethyl 4-(6-butyl-2-[3-(methyloxy)-3-oxopropyl]-8-qulnollnyl)-1-piperazinecarboxylate

To a stirred solution of 1,1-dimethylethyl 4-{6-bromo-2-[3-(methyloxy)-3-oxopropyl}-8-quinolinyl}-1-piperazinecarboxylate (for example, as prepared for intermediate 31) (2.06 g) in anhydrous THF (25 ml), under a nitrogen atmosphere, was added potassium carbonate (1.19 g), [1,1'-bis(diphenylphosphino)ferrocene)palladium (II) chloride (0.35 g) and tributylborane (commercially available, for example, from Aldrich) (1 M solution in THF, 6.5 ml). The mixture was heated at 70 °C for 5 h, then cooled to room temperature, filtered through a pad of celite and evaporated. The residue was purified by Flashmaster 11 on a 100 g silica cartridge eluting with a 0-50% EtOAc - cyclohexane gradient over 40 min. Evaporation of the solvent from appropriate fractions gave *the title compound* (1.79 g). LCMS RT = 3.89 min, ES+ve *m*/*z* 456 [M+H]⁺.

### Intermediate 33

### Methyl 3-[6-butyl-8-(1-piperazinyl)-2-quinolinyl]propanoate

To a stirred solution of 1,1-dimethylethyl 4-{6-butyl-2-[3-(methyloxy)-3-oxopropyl]-8-quinolinyl}-1-piperazinecarboxylate (for example, as prepared for intermediate 32) (1.77 g) in DCM (30 ml) was added trifluoroacetic acid (15 ml). After 30 min the mixture was evaporated, and the residue was re-dissolved in DCM (100 ml), washed with saturated aqueous sodium hydrogen carbonate solution (100 ml), dried over sodium sulphate and evaporated. The residue was applied to a MeOH preconditioned 20 g SCX-2 ion exchange cartridge, and the cartridge was eluted with MeOH, then 2M ammonia In MeOH. Evaporation of the solvent from the ammonia-containing fractions gave *the title compound* (1.28 g). LCMS RT = 2.51 min, ES+ve *m*/*z* 356 [M+H]⁺.

### Intermediate 34

### Methyl 3-(6-butyl-8-{4-[(4-{[3-(hexahydro-1H-azepin-1-yl)propyl]oxy}phenyl) acetyl]-1-piperazinyl}-2-quinollnyl)propanoate

To a stirred suspension of 2-(4-{[3-(hexahydro-1*H*-azepin-1-yl)propyl]oxy}phenyl)acetic acid hydrochloride salt (for example, as prepared for intermediate 41) (656 mg) in acetonitrile (15 ml), under a nitrogen atmosphere, was added *N*-(3-dimethylaminopropyl) *N*'-ethylcarbodiimide hydrochloride (384 mg), 1-hydroxybenzotriazole hydrate (306 mg) and Et₃N (0.55 ml). After 20 min a solution of methyl 3-[6-butyl-8-(1-piperazinyl)-2-quinolinyl]propanoate (for example, as prepared for intermediate 33) (500 mg) in acetonitrile (5 mi) was added. Stirring was continued at room temperature for 4 h, then the mixture was evaporated. The residue was partitioned between saturated aqueous sodium hydrogen carbonate solution (50 ml) and EtOAc (50 ml). The layers were separated and the aqueous phase was further extracted with EtOAc (50 ml). The combined organic extracts were washed with water (50 ml) and saturated aqueous sodium chloride (30 ml), dried over sodium sulphate and evaporated. The residue was re-evaporated from DCM to give *the title compound* (874 mg). LCMS RT = 2.91 min, ES+ve *m*/*z* 629 [M+H]⁺.

### Intermediate 35

### 3-Iodo-8-(1-piperaxinyl)quinoline

A mixture of 8-fluoro-3-iodoquinoline (as disclosed in International Patent Application WO2007039220A1, see page 16, Description 1) (1.36 g, 5 mmol) potassium carbonate (0.69 g, 5 mmol) and piperazine (3.44 g, 40 mmol) was treated with DMSO (7 ml) and the suspension was heated to 100 °C for 22.5 h. The reaction mixture was allowed to cool to room temperature and diluted with DCM (50 ml) and water. The organic solution was washed with water (2 x 50 ml). The organic phase was extracted with 4M hydrogen chloride solution (50 ml). The aqueous layer was washed with DCM (10 ml) and then added to solid potassium carbonate (30 g, 217 mmol). Water (50 ml) and DCM (50 ml) were added and the organic phase separated. The aqueous phase was extracted once more with DCM (20 ml) and the combined organic layers were dried (MgSO₄), evaporated under reduced pressure to give *the title compound* (1.3 g, 77%). LCMS RT = 2.10 min, ES+ve *m*/*z* 340 (M+H)⁺

### Intermediate 36

### 1,1-Dimethylethyl 4-(3-Iodo-8-quinolinyl)-1-piperazinecarboxylate

A solution of 3-iodo-8-(1-piperazinyl)quinoline (for example, as prepared for intermediate 35) (1.3 g, 3.8 mmol) in DCM (6 ml) and NEt₃ (1.07 ml, 7.66 mmol) was treated with di-*tert-*butyl dicarbonate (1.04 g, 4.75 mmol) and the mixture was left at room temperature for 3 days. The reaction mixture was diluted with DCM and washed 2M hydrogen chloride solution, sodium bicarbonate solution, dried (MgSO₄), evaporated under reduced pressure. The residue was diluted with DCM and applied to a silica cartridge (70 g) and purified by chromatography on Flashmaster 2, eluting with 0 to 100% EtOAc-cyclohexane over 60 min. The appropriate fractions were combined and evaporated under reduced pressure to give *the title compound* (1.45 g, 87%). LCMS RT = 3.71 min, ES+ve *m*/*z* 440 (M+H)⁺.

### Intermediate 37

### 1,1-Dlmethytethyl 4-{3-[(1E)-3-(methy)oxy)-3-oxo-1-propen-1-yl]-8-quinonyl}-1-piperazinecarboxylate

A mixture of 1,1-dimethylethyl 4-(3-iodo-8-quinolinyl)-1-piperazinecarboxylate (for example, as prepared for Intermediate 36) (555 mg, 1.26 mmol), methyl acrylate (commercially available, for example, from Aldrich) (0.345 ml, 3.8 mmol), NEt₃ (1.14 ml, 8.2 mmol), triphenylphosphine (33 mg, 0.12 mmol), palladium (II) acetate (18 mg, 0.12 mmol) in DMF (15 ml) was heated to 100 °C overnight. The mixture was concentrated under reduced pressure, and the residue was loaded on a SCX cartridge (20 g) eluting with MeOH. The appropriate fractions were combined and evaporated under reduced pressure to give *the title compound* (90 mg) LCMS RT = 3.49 min, ES+ve *m*/*z* 398 (M+H)⁺.

### Intermediate 38

### 1,1-Dimethylethyl 4-{3-[3-(methyloxy)-3-oxopropyl]-8-quinolinyl}-1-piperazinecarboxylate

A solution of 1,1-dimethylethyl 4-{3-[(1*E*)-3-(methyloxy)-3-oxo-1-propen-1-yl]-8-quinolinyil}-1-piperazinecarboxylate (for example, as prepared for Intermediate 37) (350 mg, 0.88 mmol) in EtOAc (15 ml) was hydrogenated over 10% palladium on carbon (100 mg) for 1.5 h at room temperature. Further catalyst (50 mg) was added and the mixture hydrogenated for a further 4 h. The catalyst was removed by filtration through a celite cartridge, and the filtrate was concentrated under reduced pressure to give *the title compound* (293 mg) LCMS RT = 3.14 min, ES+ve *m*/*z* 400 (M+H)⁺.

### Intermediate 39

### Methyl 3-[8-(1-piparazinyl)-3-quinolinyl]propanoate

A solution of 1,1-dimethylethyl 4-{3-[3-(methyloxy)-3-oxopropyl]-8-quinolinyl}-1-piperazinecarboxylate (for example, as prepared for Intermediate 38) (293 mg, 0.73 mmol) in DCM (10 ml) was treated with trifluoroacetic acid (3 ml) and the mixture was stirred under nitrogen at room temperature for 1 h. The reaction mixture was concentrated under reduced pressure and the residue was dissolved in toluene and re-evaporated (2 x 30 ml). The residue (330 mg) was applied to an SCX cartridge (10 g) eluting with MeOH, followed by 2N ammonia in MeOH. The appropriate fractions were combined, evaporated, and re-applied to an SCX cartridge and eluted as above to give the title compound (203 mg) LCMS RT =1.93 min, ES+ve *m*/*z* 300 (M+H)⁺.

### Intermediate 40

### Methyl (4-{[3-(hexahydro-1H-azepin-1-yl)propyl]oxy}phenyl)acetate

A mixture of di-tert-butyl azodicarboxylate (2.55 g, 11.1 mmol) and triphenylphosphine (2.91 g, 11.1 mmol) in THF (20 ml) was cooled to -20 °C to -25 °C under nitrogen. In the meantime a mixture of 3-(hexahydro-1*H*-azepin-1-yl)-1-propanol (for example, as prepared by E. L. Strogryn, J. Med. Chem. 1970, 13, 864-6, and/or is commercially available, for example, from ChemBridge) (1.80 g, 11.4 mmol) and methyl 4-hydroxyphenylacetate (commercially available, for example, from Aldrich) (1.67 g, 10.0 mmol) was suspended in THF (30 ml) and then added to the first solution after 10 min. The mixture was allowed to warm to room temperature and stirred overnight under nitrogen. The reaction mixture was partitioned between diethyl ether and brine. The aqueous layer was extracted with ether twice and the combined organic solutions were dried (MgSO₄) and evaporated under reduced pressure. The residue was applied to a SCX-2 cartridge (70 g) and eluted with MeOH, followed by 10% aqueous 0.88 ammonia in MeOH. The ammoniacal solutions were combined and evaporated. The residue (2.59 g) was purified by Flashmaster 2 chromatography on a silica cartridge (100 g) eluting with 0 to 30% MeOH (containing 1% Et₃N) - DCM over 60 min. Appropriate fractions were combined and evaporated under reduced pressure to give the title compound (1.77 g, 57%) LCMS RT = 2.08 min, ES+ve *m*/*z* 306 (M+H)⁺.

### Intermediate 41

### (4-{[3-(Hexahydro-1H-azepin-1-yl)propyl]oxy}phenyl)acetic acid, hydrochloride salt

A solution of methyl (4-{[3-(hexahydro-1H-azepin-1-yl)propyl]oxy}phenyl)acetate (for example, as prepared for Intermediate 40) (1.379 g, 4.51 mmol) in MeOH (12 ml) was treated with 2M sodium hydroxide solution (6 ml) and the resulting mixture was stirred at ambient temperature overnight. The solvent was evaporated giving a residue that was partitioned between dilute hydrochloric acid (9 ml of 2M diluted to 25 ml with water) and 3:1 chloroform-iso-propanol (25 ml). The aqueous layer was extracted further with the chloroform-*iso*-propanol mixture (4 x 25 ml). The combined extracts were dried over anhydrous sodium sulphate and the solvent was evaporated to give *the title compound* as a white solid (1.4646 g). LCMS RT = 1.96 min, ES+ve *m*/*z* 292 [M+H]⁺.

### Examples

### Example 1

### 3-[8-{4-[2-(4{[3-(Hexahydro-1H-azepin-1-yl)propyl]oxy}phenyl)ethyl]-1-piperazinyl}-6-(phenylmethyl)-2-quinolinyl]propanoic acid, formate salt

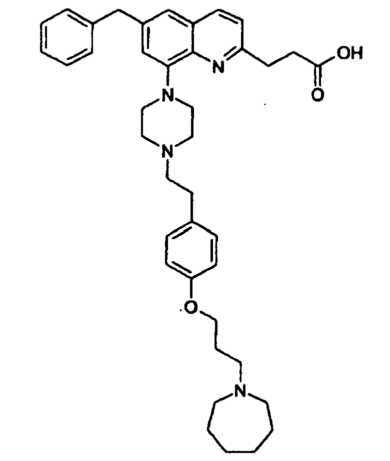

A solution of methyl 3-[8-{4-[2-(4-{[3-(hexahydro-1*H*-azepin-1-yl)propyl]oxy}phenyl)ethyl]-1-piperazinyl}6-(phenylmethyl)-2-quinolinyl]propanoate (for example, as prepared for Intermediate 9) (32 mg) in MeOH (5 ml) was treated with 2N sodium hydroxide solution (1 ml) and the resulting mixture was heated at 65 °C for 1 h. The reaction mixture was treated with 2N hydrochloric acid (1 ml) and the solvent was evaporated. The residue was dissolved in MeOH- DMSO (1:1, 1 ml) and purified by MDAP. Evaporation of the solvent from appropriate fractions gave the *title compound* (11.5 mg). LCMS RT = 2.39 min, ES+ve *m*/*z* 635 [M+H]⁺. ¹H NMR δ (400 MHz, MeOD) 1.71 (s, 4 H), 1.89 (s, 4 H), 2.15 - 2.22 (m, 2 H), 2.80 (t, *J*=7.03 Hz, 2 H), 2.90 - 2.97 (m, 2 H), 3.02 - 3.09 (m, 2 H), 3.18 - 3.23 (m, 4 H), 3.23 - 3.28 (m, *J*=7.03 Hz, 2 H), 3.27 - 3.29 (m, 2 H), 3.31 - 3.34 (m, 4 H), 3.48 (s, 4 H), 4.02 - 4.08 (m, 4 H), 6.89 (d, *J*=8.53 Hz, 2 H), 7.03 (d, *J*=2.00 Hz, 1 H), 7.16 - 7.27 (m, 7 H), 7.28 - 7.30 (m, 1 H), 7.35 (d, *J*=8.53 Hz, 1 H), 8.02 (d, *J*=8.53 Hz, 1 H), 8.52 (s, 1 H).

### Example 2

### 3-(6-Ethyl-8-{4-[2-(4-([3-(hexahydro-1H-azepin-1-yl)propyl]oxy}phenyl)ethyl]-1-piperazinyl}-2-quinolinyl)propanoic acid, formate salt

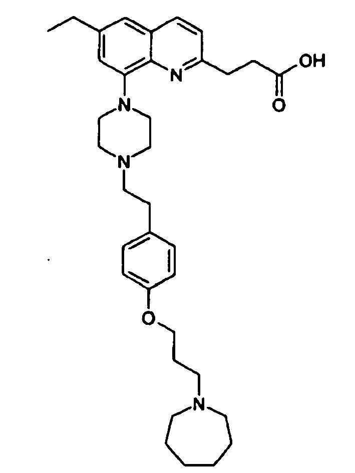

A solution of methyl 3-(6-ethyl-8-{4-[2-(4-{[3-(hexahydro-1*H*-azepin-1-yl)propyl]oxy}phenyl)ethyl]-1-piperazinyl}-2-quinolinyl)propanoate (for example, as prepared for Intermediate 12) in MeOH (5 ml) was treated with 2N sodium hydroxide solution (1 ml) and the resulting mixture was heated at 65 °C with stirring for 2 h. The reaction mixture was treated with 2N hydrochloric acid (1 ml) and the solvent was evaporated giving a residue that was suspended in MeOH-DMSO (1:1, 2 ml). The suspension was filtered and the filtrate was purified by MDAP. Evaporation of the solvent from appropriate fractions gave the *title compound* (35 mg). LCMS RT = 2.10 min, ES+ve *m*/*z* 573 [M⁺H]⁺, 287 (M/2+H)⁺.

### Example 3

### 3-(8-{4-[2-(4-{[3-(Hexahydro-1H-azepin-1-yl)propyl]oxy}phenyl)ethyl]-1-piperazinyl)-2-quinolinyl)propanoic acid, formic acid (1:2)

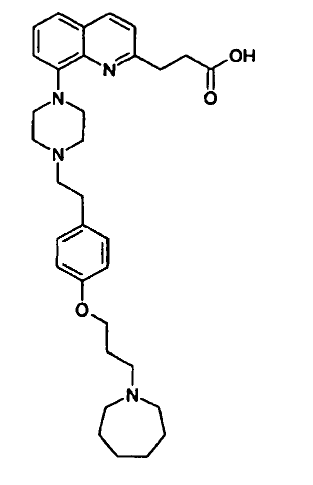

(a) A mixture of methyl 3-(8-{4-[2-(4-{[3-(hexahydro-1*H*-azepin-1-yl)propyl]oxy}phenyl)ethyl}-1-piperazinyl}-2-quinolinyl)propanoate and ethyl 3-(8-{4-[2-(4-{[3-(hexahydro-1*H*-azepin-1-yl)propyl]oxy}phenyl)ethyl}-1-piperazinyl}-2-quinolinyl)propanoate (for example, as prepared for Intermediate 24) (370 mg) in THF (10 ml) was treated with sodium hydroxide solution (2M, 10 ml) and the mixture was stirred overnight at room temperature. Acetic acid (10 ml) was added and the mixture was concentrated. The residue was applied to SCX-2 cartridge (10 g), washed with MeOH and eluted with methanolic ammonia. Product was found in all fractions so they were combined and evaporated. The crude product was re-dissolved in MeOH (5 ml) and treated again with sodium hydroxide solution (2 ml) and stirred for 4 h. Acetic acid (2 ml) was added and the mixture concentrated. The residue was applied to a SCX-2 cartridge (20 g) eluting with MeOH first, and then with ammonia in MeOH. The basic fractions were combined and evaporated to give the free base of *the tit*/*e compound* (347 mg). LCMS RT = 2.00 min, ES+ve *m*/*z* 545 (M+H)⁺, 273 (M/2+H)⁺. A portion of the free base was further purified by MDAP to give the title compound LCMS RT = 1.87 min, ES+ve *m*/*z* 545 (M+H)⁺, 273/274 (M/2+H)⁺.
(b) 3-(8-{4-[2-(4-{[3-(Hexahydro-1*H*-azepin-1-yl)propyl]oxy}phenyl)ethyl]-1-piperazinyl}-2-quinolinyl)propanoic acid, formic acid salt
   Methyl 3-(8-{4-[2-(4-{[3-(hexahydro-1*H*-azepin-1-yl)propyl]oxy}phenyl)ethyl]-1-piperazinyl}-2-quinolinyl)propanoate (for example, as prepared for Intermediate 30) (34 mg) was dissolved in MeOH (8 ml) and 2M sodium hydroxide solution (1 mi) was added. The mixture was stirred at 70 °C for 4 h at which time 2M hydrochloric acid (1 ml) was added. The mixture was filtered and the filtrate was purified by MDAP to give the *title compound* (23 mg). LCMS RT= 2.06 min, ES+ve *m*/*z* 545 [M+H]⁺.

### Example 4

### (2E)-3-(8-{4-[2-(4-{[3-(Hexahydro-1H-azepin-1-yl)propyl]oxy}phenyl)ethyl]-1-piperazinyl}-2-quinolinyl)-2-propenoic acid-formic acid (1:1)

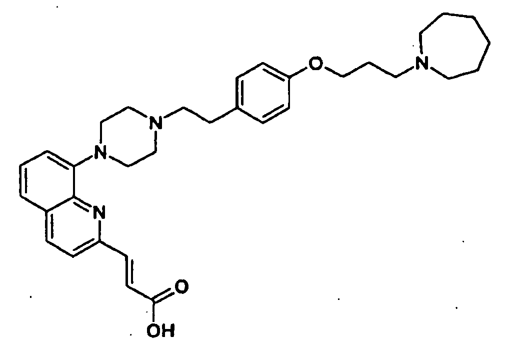

Methyl 3-(8-{4-[2-(4-{[3-(hexahydro-1*H*-azepin-1-yl)propyl]oxy}phenyl)ethyl]-1-piperazinyl}-2-quinolinyl)-2-propenoate - formic acid (1:1) (for example, as prepared for Intermediate 23) (15.2 mg, 0.0252 mmol) was dissolved in anhydrous THF (1 ml) and treated with 2N NaOH (1 ml). The reaction mixture was stirred at room temperature for two days. The reaction was quenched with acetic acid (0.5 ml) and loaded on to a 2 g SCX-2 cartridge which was eluted with MeOH, followed by 2 M methanolic ammonia. Some product eluted in the MeOH fraction. Therefore, both the MeOH and MeOH/ammonia fractions were combined and evaporated. The crude material was purified by MDAP. Appropriate fractions were combined and evaporated to give *the title compound* (2.9 mg). LCMS RT = 2.15 min, ES+ve *m*/*z* 272 (M/2+H)⁺. 543 (M+H)⁺.

### Example 6

### 3-(8-{4-[2-{[4-{[3-(hexahydro-1H-azepin-1-yl)propyl)oxy}phenyl)ethyl]-1-piperazinyl}-3-quinolinyl)propanoic acid, formic acid salt

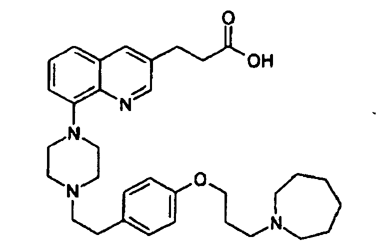

The compound of Example 5 was prepared by a similar method to that described for the compound of Example 1. Reaction of 2-(4-{[3-(hexahydro-1*H*-azepin-1-yl)propyl]oxy}phenyl)ethyl methanesulfonate (for example, as prepared for Intermediate 15) with methyl 3-[8-(1-piperazinyl)-3-quinolinyl]propanoate (for example, as prepared for Intermediate 39) was carried out as described for Intermediate 9, followed by ester hydroylsis of the product, carried out according to the method described for Example 1.

### Example 6

### 3-(6-Butyl-8-{4-[2-(4-{[3-(hexahydro-1H-azepin-1-yl)propyl]oxy}phenyl)ethyl]-1-piperazinyl}-2-quinolinyl)propanoic acid, formate salt

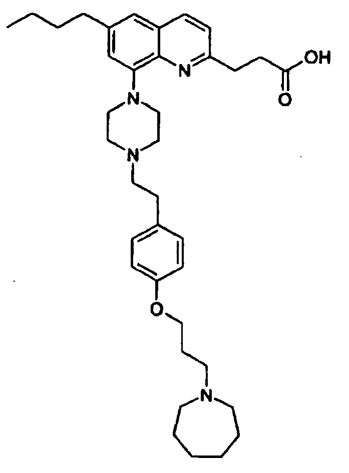

To a stirred solution of methyl 3-(6-butyl-8-{4-[(4-{[3-(hexahydro-1*H*-azepin-1-yl)propyl]oxy}phenyl)acetyl]-1-piperazinyl}-2-quinolinyl)propanoate (for example, as prepared for intermediate 34) (867 mg) in anhydrous THF (15 ml), under a nitrogen atmosphere was added carbonylhydridotris(triphenylphosphine)rhodium (1) (63 mg) and diphenylsilane (0.54 ml). After stirring for 1 h further amounts of carbonylhydridotris (triphenylphosphine)rhodium (1) (25 mg) and diphenylsilane (0.22 ml) were added. The mixture was stirred for 30 min then diluted with ether (30 ml) and extracted with 1 M hydrochloric acid (40 ml). The aqueous layer was basified by the addition of saturated aqueous sodium carbonate then extracted with EtOAc (2 x 30 ml). The combined EtOAc extracts were dried over sodium sulphate and evaporated. The residue was re-evaporated from DCM then dissolved in MeOH (8 ml) and treated with 2M sodium hydroxide (2 ml). The mixture was heated at 70 °C for 1 h and then cooled to room temperature. The mixture was adjusted to pH 8 by the addition of 2M hydrochloric acid, diluted with water (40 ml) and extracted with EtOAc (3 x 30 ml). The combined organic extracts were dried over sodium sulphate and evaporated. The residue was re-evaporated from DCM then dissolved in MeOH - DMSO (1:1) and purified by MDAP. Evaporation of the solvent from appropriate fractions gave *the title compound* (336 mg). LCMS RT = 2.38 min, ES+ve *m*/*z* 601 [M+H]⁺.

### Biological Data

Compounds of the invention may be tested for *in vitro* biological activity for example in accordance with the following or similar assays.
**H1 receptor cell line generation and FLIPR assay protocol**

### 1. Generation of histamine H1 cell line

The human H1 receptor is cloned using known procedures described in the literature *[*Biochem. Biophys. Res. Commun., 201(2):894 (1994)]. Chinese hamster ovary (CHO) cells stably expressing the human H1 receptor are generated according to known procedures described in the literature *[*Br. J. Pharmacol., 117(6):1071 (1996)].

### Histamine H1 functional antagonist assay: Determination of functional pKi values

The histamine H1 cell line is seeded into non-coated black-walled clear bottom 384-well tissue culture plates in alpha minimum essential medium (Gibco/Invitrogen, cat no. 22561-021), supplemented with 10% dialysed foetal calf serum (Gibco/Invitrogen cat no. 12480-021) and 2 mM L-glutamine (Gibco/Invitrogen cat no 25030-024) and is maintained overnight at 5% CO₂, 37 °C.

Excess medium is removed from each well to leave 10 µl. 30 µl loading dye (250 µM Brilliant Black, 2 pM Fluo-4 diluted in Tyrodes buffer + probenecid (145 mM NaCi, 2.5 mM KCI, 10 mM HEPES, 10 mM D-glucose, 1.2 mM MgCh₂, 1.5 mM CaCl₂, 2.5 mM probenecid, pH adjusted to 7.40 with NaOH 1.0 M)) is added to each well and the plates are incubated for 60 min at 5% CO₂, 37 °C.

10 µl of test compound, diluted to the required concentration in Tyrodes buffer + probenecid (or 10 µl Tyrodes buffer + probenecid as a control) is added to each well and the plate is incubated for 30 min at 37 °C, 5% CO₂. The plates are then placed into a FLIPR™ (Molecular Devices, UK) to monitor cell fluorescence (λₑₓ = 488 nm, λ_{EM} = 540 nm) in the manner described in Sullivan *et al.,* (In: Lambert DG (ed.), Calcium Signaling Protocols, New Jersey: Humana Press, 1999, 125-136) before and after the addition of 10 µl histamine at a concentration that results In the final assay concentration of histamine being EC₈₀.

Functional antagonism is indicated by a suppression of histamine induced increase in fluorescence, as measured by the FLIPR™ system (Molecular Devices). By means of concentration effect curves, functional affinities are determined using standard pharmacological mathematical analysis.

### Histamine H1 functional antagonist assay: Determination of antagonist pA2 and duration

The histamine H1 receptor expressing CHO cells are seeded into non-coated black-walled clear bottom 96-well tissue culture plates as described above.

Following overnight culture, growth tedium is removed from each well, washed with 200 µl phosphate buffered saline (PBS) and is replaced with 50 µl loading dye (250 µM Brilliant Black, 1 µM Flux-4 diluted in Tyrodes buffer + probenecid (145 mM NaCl, 2.5 mM KCI, 10mM HEPES, 10mM D-glucose, 1.2 mM MgCl₂, 1.5 mM CaCl₂. 2.5 mM probenecid, pH adjusted to 7.40 with NaOH 1.0 M)). Cells are incubated for 45 min at 37 **°**C**.** The loading buffer is removed and the cells are washed as above, and 90 µl of Tyrodes buffer + probenecid is added to each well. 10 µl of test compound, diluted to the required concentration in Tyrodes buffer + probenecid (or 10 µl Tyrodes buffer + probenecid as a control) is added to each well and the plate is incubated for 30 min at 37 °C**.** 5% CO₂.

The plates are then placed into a FLlPR™ (Molecular Devices, UK) to monitor cell fluorescence (λₑₓ = 488 nm, λ_{EM} = 540 nm) in the manner described in Sullivan *et al.,* (In: Lambert DG (ed.), Calcium Signaling Protocols, New Jersey: Humana Press, 1999, 125-136) before and after the addition of 50 µl histamine over a concentration range of 1 mM - 0.1 nM. The resultant concentration response curves are analysed by non-linear regression using a standard four parameter logistic equation to determine the histamine EC₅₀, the concentration of histamine required to produce a response of 50% of the maximum response to histamine. The antagonist pA2 is calculated using the following standard equation: pA2 = log(DR-1)-log[B] where DR = dose ratio, defined as EC₅₀ antagonist-treated/EC₅₀control and [B] = concentration of antagonist.

To determine the antagonist duration, cells are cultured overnight in non-coated black-walled clear bottom 96-well tissue culture plates, are washed with PBS and are incubated with a concentration of antagonist chosen to give an approximate DR in the range 30 - 300. Following the 30 min antagonist incubation period, the cells are washed two or three times with 200 µl of PBS and then 100 µl Tyrodes buffer is added to each well to initiate antagonist dissociation. Following incubation for predetermined times, typically 30 - 270 min at 37 °C, the cells are then washed again with 200 µl PBS and are incubated with 100 µl Tyrodes buffer containing Brilliant Black, probenecid and Fluo-4 for 45 min at 37 °C, as described above. After this period, the cells are challenged with histamine in the FLIPR™ as described above. The dose ratio at each time point is used to determine the fractional H1 receptor occupancy by the following equation: fractional receptor occupancy = (DR-1)/DR. The decrease in receptor occupancy over time approximates to a straight line and is analysed by linear regression. The slope of this straight line fit is used as an index of the dissociation rate of the antagonist. The dose ratios for antagonist treated cells and for antagonist treated and washed cells at each time point are used to calculate a relative dose ratio (rel DR) which is also used as an index of antagonist duration. Antagonists with long duration of action produce rel DR values close to 1, and antagonists with short duration of action produce rel DR values that approaches the dose ratio value obtained for antagonist treatment alone.

### 2. H3 receptor cell line generation, membrane preparation and functional GTP_{Y}S assay protocols

### Generation of histamine H3 cell line

The histamine H3 cDNA is isolated from its holding vector, pCDNA3.1 TOPO (InVitrogen), by restriction digestion of plasmid DNA with the enzymes BamH1 and Not-1 and is ligated into the inducible expression vector pGene (InVitrogen) digested with the same enzymes. The GeneSwitch™ system (a system where in transgene expression is switched off in the absence of an inducer and switched on in the presence of an inducer) is performed as described in US Patents: 5,364,791; 5,874,534; and 5,935,934. Ligated DNA is transformed into competent DH5a E. coli host bacterial cells and is plated onto Luria Broth (LB) agar containing Zeocin™ (an antibiotic which allows the selection of cells expressing the *sh ble* gene which is present on pGene and pSwitch) at 50 µgml⁻¹. Colonies containing the re-ligated plasmid are identified by restriction analysis. DNA for transfection into mammalian cells is prepared from 250 ml cultures of the host bacterium containing the pGeneH3 plasmid and is isolated using a DNA preparation kit (Qiagen Midi-Prep) as per manufacturers guidelines (Qiagen).

CHO K1 cells previously transfected with the pSwitch regulatory plasmid (InVitrogen) are seeded at 2x10⁶ cells per T75 flask in Complete Medium, containing Hams F12 (GIBCOBRL, Life Technologies) medium supplemented with 10% v/v dialysed foetal bovine serum, L-glutamine, and hygromycin (100 µgml⁻¹), 24 h prior to use. Plasmid DNA is transfected into the cells using Lipofectamine plus according to the manufacturer's guidelines (InVitrogen). 48 h post transfection, cells are placed into complete medium supplemented with 500 µgml⁻¹ Zeocin™.

10-14 days post selection, 10 nM Mifepristone (InVitrogen) is added to the culture medium to induce the expression of the receptor. 18 h post induction, cells are detached from the flask using ethylenediamine tetra-acetic acid (EDTA; 1:5000; InVitrogen), following several washes with PBS, pH 7.4 and are resuspended in Sorting Medium containing Minimum Essential Medium (MEM), without phenol red, and are supplemented with Earles salts and 3% Foetal Clone II (Hyclone). Approximately 1x10⁷ cells are examined for receptor expression by staining with a rabbit polyclonal antibody, 4a, raised against the N-terminal domain of the histamine H3 receptor, are incubated on ice for 60 min, followed by two washes in sorting medium. Receptor bound antibody is detected by incubation of the cells for 60 min on ice with a goat anti rabbit antibody, conjugated with Alexa 488 fluorescence marker (Molecular Probes). Following two further washes with Sorting Medium, cells are filtered through a 50 µm Filcon™ (BD Biosciences) and then are analysed on a FACS Vantage SE Flow Cytometer fitted with an Automatic Cell Deposition Unit Control cells are non-induced cells treated in a similar manner. Positively stained cells are sorted as single cells into 96-well plates, containing Complete Medium containing 500 µgml⁻¹ Zeocin™ and are allowed to expand before reanalysis for receptor expression via antibody and ligand binding studies. One clone, 3H3, is selected for membrane preparation.

### Membrane preparation from cultured cells

All steps of the protocol are carried out at 4 °C and with pre-cooled reagents. The cell pellet is resuspended in 10 volumes of homogenisation buffer (50 mM *N*-2-hydroxyethylpiperazine-*N*-2-ethanesulfonic acid (HEPES), 1 mM ethylenediamine tetra-acetic acid (EDTA), pH 7.4 with KOH, supplemented with 10-⁸ M leupeptin (acetyl-leucyl-leucyl-arginal; Sigma L2884), 25 µgm⁻¹ bacitracin (Sigma B0125), 1 mM phenylmethylsulfonyl fluoride (PMSF) and 2x10⁻⁶ M pepstain A (Sigma)). The cells are then homogenised by 2 x 15 second bursts in a 1 litre glass Waring blender, followed by centrifugation at 500 g for 20 min. The supernatant is then spun at 48,000 g for 30 min. The pellet is resuspended in homogenisation buffer (4x the volume of the original cell pellet) by vortexing for 5 sec, followed by homogenisation in a Dounce homogeniser (10-15 strokes). At this point the preparation is aliquoted into polypropylene tubes and stored at -80 °C.

### Histamine H3 functional antagonist assay

For each compound being assayed, in a solid white 384 well plate, is added:-
(a) 0.5 µl of test compound diluted to the required concentration in DMSO (or 0.5 µl DMSO as a control);
(b) 30 µl bead/membrane/GDP minx which is prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20 mM *N*-2-hydroxyethylpiperazine-*N'*-2-ethanesulfonic acid (HEPES) + 100 mM NaCl + 10 mM MgCl₂, pH 7.4 NaOH) to give a final volume of 30 µl which contains 5 µg protein, 0.25 mg bead per well and 10 µM final assay concentration of guanosine 5' diphosphate (GDP) (Sigma, diluted in assay buffer) incubating at room temperature for 60 min on a roller;
(c) 15 µl 0.38 nM [³⁵S]-GTPγS (Amersham; Radioactivity concentration = 37 MBqml⁻¹; Specific activity = 1160 Cimmol⁻¹), histamine (at a concentration that results in the final assay concentration of histamine being EC₈₀).

After 2-6 h, the plate is centrifuged for 5 min at 1500 rpm and counted on a Viewlux counter using a 613/55 filter for 5 minplate⁻¹. Data is analysed using a 4-parameter logistic equation. Basal activity is used as minimum, i.e. histamine not added to well.

### CNS penetration by bolus administration

Compounds are dosed intravenously at a nominal dose level of 1 mg/kg to male CD Sprague Dawley rats. Compounds are formulated in 5% DMSO/45% PEG200/50% water. Blood samples are taken under terminal anaesthesia with isoflurane at 5 min post-dose and the brains are also removed for assessment of brain penetration. Blood samples are taken directly into heparinised tubes. Blood samples are prepared for analysis using protein precipitation and brain samples are prepared using extraction of drug from brain by homogenisation and subsequent protein precipitation. The concentration of parent drug in blood and brain extracts is determined by quantitative LC-MS/MS analysis using compound-specific mass transitions.

### Results

The compounds of Examples 1 to 6 were tested in the above or similar assays/methods and showed:
(i) an average pK, (pK_{b}) greater than 7.0.
(ii) an average pKₗ (pK_{b}) at H3 of greater than 8.0.
(iii) Example 5 had an average pA2 value of greater than approximately 7.0. Examples 1, 2, 3a and 6 had average pA2 values of, greater than approximately 8.0.
(iv) Examples 1, 2 and 3 demonstrated low CNS penetration. Other compounds are predicted to have low CNS penetration.

## Claims

1. A compound of formula (I) 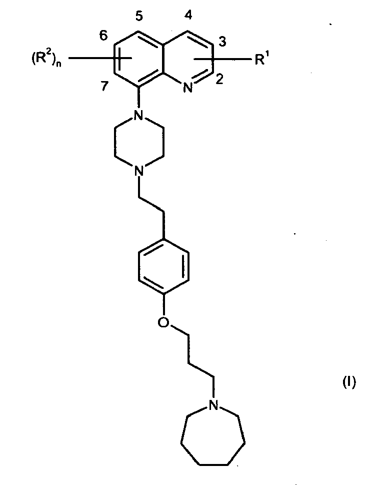 wherein
the quinolinyl ring is substituted in the 2 or 3 position by R¹ and is optionally substituted in the 5, 6, or 7 position by R²;
R¹ represents -CH₂CH₂COOH, -CH=C(CH₃)COOH or -CH=CHCOOH;
R² represents C₁₋₆alkyl, aryl or -C₁₋₆alkylaryl;
n represents 0 or 1; and
a salt thereof.

2. A compound according to claim 1, or a salt thereof, where the quinolinyl ring is substituted in the 2 position by R¹.

3. A compound according to claim 1 or claim 2, or a salt thereof, wherein R¹ represents -CH₂CH₂COOH.

4. A compound according to any of claims 1 to 3, or a salt thereof, wherein R² represents C₁₋₄alkyl, phenyl, or -C₁₋₄alkylphenyl.

5. A compound as in claim 1 which is 3-[8-{4-[2-(4-{[3-(hexahydro-1*H*-azepin-1-yl)propyl] oxy}phenyl)ethyl]-1-piperazinyl}-6-(phenylmethyl)-2-quinolinyl]propanoic acid, or a salt thereof.

6. A compound according to any of claims 1 to 5, or a pharmaceutically acceptable salt thereof.

7. A compound according to any of claims 1 to 5, or a pharmaceutically acceptable salt thereof for use in therapy.

8. A compound according to claim 7, or a pharmaceutically acceptable salt thereof for use in the treatment of inflammatory and/or allergic diseases.

9. A compound according to claim 7, or a pharmaceutically acceptable salt thereof for use in the treatment of allergic rhinitis.

10. A composition which comprises a compound as defined in any of claims 1 to 5, or a pharmaceutically acceptable salt thereof and one or more pharmaceutically acceptable carriers and/or excipients.

11. A combination comprising a compound or a pharmaceutically acceptable salt thereof as defined in any of claims 1 to 5 and one or more other therapeutic compounds.

12. The use of a compound as defined in any of claims 1 to 5, or a pharmaceutically acceptable salt thereof in the manufacture of a medicament for the treatment or prophylaxis of inflammatory and/or allergic diseases. ,

13. The use according to claim 12, in which the disease is allergic rhinitis.

## Patentansprüche

1. Verbindung der Formel (I): 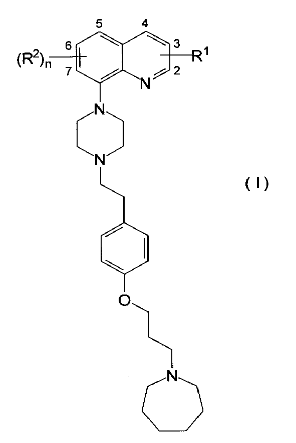 wobei
der Chinolinring in der 2- oder 3-Position mit R¹ substituiert ist und gegebenenfalls in der 5-, 6- oder 7-Position mit R² substituiert ist;
R¹ -CH₂CH₂COOH, -CH=C(CH₃)COOH oder -CH=CHCOOH darstellt;
R² C₁₋₆-Alkyl, Aryl oder -C₁₋₆-Alkylaryl darstellt;
n 0 oder 1 darstellt; und
ein Salz davon.

2. Verbindung gemäss Anspruch 1 oder ein Salz davon, wobei der Chinolinring in der 2-Position mit R¹ substituiert ist.

3. Verbindung gemäss Anspruch 1 oder Anspruch 2 oder ein Salz davon, wobei R¹ -CH₂CH₂COOH darstellt.

4. Verbindung gemäss einem der Ansprüche 1 bis 3 oder ein Salz davon, wobei R² C₁₋₄-Alkyl, Phenyl oder -C₁₋₄-Alkylphenyl darstellt.

5. Verbindung wie in Anspruch 1, nämlich 3-[8-(4-[2-(4-{[3-(Hexahydro-1H-azepin-1-yl)propyl]oxy}phenyl)-ethyl]-1-piperazinyl)-6-(phenylmethyl)-2-chinolinyl]-propansäure, oder ein Salz davon.

6. Verbindung gemäss einem der Ansprüche 1 bis 5 oder ein pharmazeutisch annehmbares Salz davon.

7. Verbindung gemäss einem der Ansprüche 1 bis 5 oder ein pharmazeutisch annehmbares Salz davon zur Verwendung in der Therapie.

8. Verbindung gemäss Anspruch 7 oder ein pharmazeutisch annehmbares Salz davon zur Verwendung in der Behandlung von entzündlichen und/oder allergischen Erkrankungen.

9. Verbindung gemäss Anspruch 7 oder ein pharmazeutisch annehmbares Salz davon zur Verwendung in der Behandlung von allergischer Rhinitis.

10. Zusammensetzung, die eine Verbindung, wie in einem der Ansprüche 1 bis 5 definiert, oder ein pharmazeutisch annehmbares Salz davon und einen oder mehrere pharmazeutisch annehmbare Träger und/oder Exzipienten umfasst.

11. Kombination, umfassend eine Verbindung oder ein pharmazeutisch annehmbares Salz davon, wie in einem der Ansprüche 1 bis 5 definiert, und eine oder mehrere andere therapeutische Verbindungen.

12. Verwendung einer Verbindung, wie in einem der Ansprüche 1 bis 5 definiert, oder eines pharmazeutisch annehmbaren Salzes davon bei der Herstellung eines Medikaments zur Behandlung oder Prophylaxe einer entzündlichen und/oder allergischen Erkrankung.

13. Verwendung gemäss Anspruch 12, wobei die Erkrankung allergische Rhinitis ist.

## Revendications

1. Composé de formule (I) 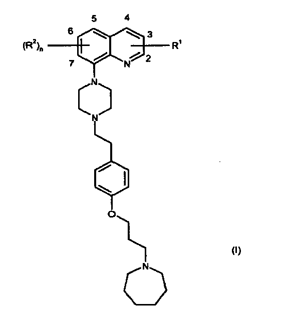 dans laquelle
le cycle quinolinyle est substitué en position 2 ou 3 par R¹ et est facultativement substitué en position 5, 6 ou 7 par R² ;
R¹ représente -CH₂CH₂COOH, -CH=C(CH₃)COOH ou -CH=CHCOOH ;
R² représente un alkyle en C₁ à C₆, un aryle ou un (alkyl en C₁ à C₆)aryle ;
n représente 0 ou 1 ; et
un sel de celui-ci.

2. Composé selon la revendication 1, ou sel de celui-ci, où le cycle quinolinyle est substitué en position 2 par R¹.

3. Composé selon la revendication 1 ou la revendication 2, ou sel de celui-ci, dans lequel R¹ représente -CH₂CH₂COOH.

4. Composé selon l'une quelconque des revendications 1 à 3, ou sel de celui-ci, dans lequel R² représente un alkyle en C₁ à C₄, un phényle ou un (alkyl en C₁ à C₄)phényle.

5. Composé selon la revendication 1, qui est l'acide 3-[8-{4-[2-(4-{[3-(hexahydro-1*H*-azépin-1-yl)-propyl]oxy}phényl)éthyl]-1-pipérazinyl]-6-(phényl-méthyl)-2-quinolinyl]propanoïque, ou l'un de ses sels.

6. Composé selon l'une quelconque des revendications 1 à 5, ou l'un de ses sels pharmaceutiquement acceptables.

7. Composé selon l'une quelconque des revendications 1 à 5, ou l'un de ses sels pharmaceutiquement acceptables, pour un usage en thérapie.

8. Composé selon la revendication 7, ou l'un de ses sels pharmaceutiquement acceptables, pour un usage dans le traitement des maladies inflammatoires et/ou allergiques.

9. Composé selon la revendication 7, ou l'un de ses sels pharmaceutiquement acceptables, pour un usage dans le traitement de la rhinite allergique.

10. Composition comprenant un composé défini dans l'une quelconque des revendications 1 à 5, ou l'un de ses sels pharmaceutiquement acceptables, et un ou plusieurs supports et/ou excipients pharmaceutiquement acceptables.

11. Combinaison comprenant un composé ou l'un de ses sels pharmaceutiques acceptables selon l'une quelconque des revendications 1 à 5 et un ou plusieurs autres composés thérapeutiques.

12. Utilisation d'un composé défini dans l'une quelconque des revendications 1 à 5, ou de l'un de ses sels pharmaceutiquement acceptables, dans la fabrication d'un médicament destiné au traitement ou à la prophylaxie des maladies inflammatoires et/ou allergiques.

13. Utilisation selon la revendication 12, dans laquelle la maladie est la rhinite allergique.
